# EUROPEAN PATENT APPLICATION

(11) **EP 4 835 078 A1**
(43) Date of publication of application: **04.11.2026**
(21) Application number: 26167328.9
(22) Date of filing: 24.03.2026
(51) Int. Cl.: C12N 15/113, A61K 31/7088

(54) **OLIGONUCLEOTIDE COMPOUND, MEDICAMENT, AND USE THEREOF**

(30) Priority: 30.04.2025 CN 202510568765
(71) Applicant: Redfield Pharmaceutical Inc., Guangzhou, Guangdong 510000 (CN); Redfield Biotech Limited, Nanjing, Jiangsu 210000 (CN); Redfield Pharma Limited, Hong Kong 999077 (HK)
(72) Inventor: LI, Wenquan, Guangzhou, 510000 (CN); HU, Xionglin, Guangzhou, 510000 (CN); CHEN, Lulu, Guangzhou, 510000 (CN); LIU, Sanxia, Guangzhou, 510000 (CN); ZHOU, Tong, Guangzhou, 510000 (CN); SHAO, Yali, Guangzhou, 510000 (CN); CHRISTOPHER SHENG, Xiaoning, Guangzhou, 510000 (CN)
(74) Representative: Russell, Tim

(57) **Abstract**

An oligonucleotide compound, a medicament, and a use thereof are provided herein. The The oligonucleotide compound includes at least one carbocyclic nucleoside of formula (I), a nucleic acid base sequence of each of the at least one oligonucleotide compound is as shown in sequence identity 1 (SEQ ID NO.1), 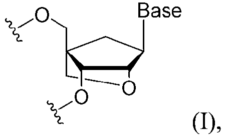 where Base is a heterocyclic base, and 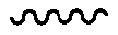 indicates a linking site. This oligonucleotide compound has excellent structural stability, specific targeting, and biological activity, and can reduce expression levels of HBV RNA, HBV DNA, and related proteins, which is beneficial for the prevention and treatment of HBV and HDV-related diseases or symptoms.

## Description

### TECHNICAL FIELD

This disclosure relates to the field of pharmaceutical technology, and in particular to an oligonucleotide compound, a medicament, and a use thereof.

### BACKGROUND

Chronic hepatitis B (CHB) is caused by infection with the hepatitis B virus (HBV). The global impact of this disease is well-documented and it is a public health issue urgently needing resolution. The World Health Organization (WHO) has compiled statistics showing that as many as 250 million people worldwide suffer from CHB, accounting for approximately 3.5% of the world's total population.

Hepatitis D virus (HDV) is a defective virus; its replication, antigen expression, and infection of hepatocytes all require the assistance of HBV. Compared to other hepatitis virus infections, HDV infection is more likely to progress to chronic hepatitis, increasing the risk of cirrhosis, liver decompensation, hepatocellular carcinoma (HCC), and liver disease-related death. It is a serious viral infection of the liver. Chronic hepatitis D is a serious viral hepatitis caused by co-infection with HDV and HBV, or by infection with HDV on top of HBV infection. HBV-infected individuals with concurrent hepatitis D often experience faster disease progression and a worse prognosis.

Given the severity of HBV infection, despite continuous research and development of new drugs and improvements in the efficacy of antiviral medications in recent years, complete cure of CHB remains a clinical challenge. While interferon has shown some effectiveness in HBV treatment, its low response rate and numerous adverse reactions are significant negative impacts. Nucleoside (nucleotide) analogues (NAs) for HBV treatment have long treatment courses and insufficient efficacy, and may be accompanied by a high risk of viral resistance mutations. With advancements in clinical technology, combined immunotherapy and NAs have become the preferred treatment option for CHB. In the current stage where no new radical cure is available, most studies have found that sequential/combined use of NAs and pegylated interferon (Peg-IFN), two drugs with different mechanisms of action, can improve treatment efficacy. The 2019 guidelines also indicate that for the advantageous population of CHB patients treated with NAs, combining Peg-IFN-α can achieve clinical cure. Although the combined use of NAs and Peg-IFN has a greater therapeutic advantage, HBsAg seroconversion remains difficult, and the clinical cure rate is still very low (not exceeding 7%). Therefore, there is a need in this field to discover and develop new anti-HBV therapies.

Antisense technology is becoming an effective means of reducing the expression of specific gene products. The difference between antisense therapy and nucleoside therapy is that antisense therapy can directly target transcripts of HBV DNA, thereby reducing serum hepatitis B e antigen (HBeAg) and hepatitis B surface antigen (HBsAg) levels. Due to the multiple overlapping transcripts produced during HBV infection, including pregenomic transcripts (pg RNA), a single antisense oligonucleotide also has the potential to reduce HBV DNA other than HBeAg and HBsAg, providing new insights for the treatment of HBV and HDV.

### SUMMARY

In view of this, the disclosure provides an oligonucleotide compound, a medicament, and an application thereof for the prevention and treatment of HBV and/or HDV-related diseases or symptoms.

In a first aspect, the disclosure provides an oligonucleotide compound. The oligonucleotide compound includes at least one carbocyclic nucleoside of formula (I), a nucleic acid base sequence of each of the at least one oligonucleotide compound is as shown in sequence identity 1 (SEQ ID NO.1), 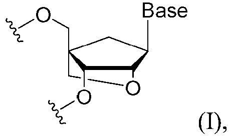 where Base is a heterocyclic base, and 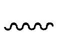 indicates a linking site.

In a second aspect, the disclosure provides a medicament including the oligonucleotide compound described in the first aspect or a pharmaceutically acceptable salt thereof.

In a third aspect, the disclosure provides a use of the oligonucleotide compound described in the first aspect or the pharmaceutically acceptable salt thereof, or the medicament described in the second aspect, in the preparation of medicaments for the prevention and/or treatment of HBV and/or HDV-related diseases or symptoms.

The oligonucleotide compound provided in the disclosure can specifically target transcripts of HBV DNA, reduce serum HBeAg, HBsAg, and HBV DNA levels. Simultaneously, the carbocyclic nucleoside can improve the resistance to nucleases and thermal stability of the oligonucleotide compound, increase its base pairing affinity, promote binding with complementary mRNA, improve specificity and targeting ability, and enhance its biological activity and therapeutic effect, which is beneficial for the prevention and treatment of HBV and/or HDV-related diseases or symptoms.

### BRIEF DESCRIPTION OF THE DRAWINGS

To more clearly illustrate technical solutions in embodiments of the present disclosure or the prior art, the drawings used in the description of the embodiments or the prior art will be briefly introduced below. The specific embodiments described herein are merely illustrative of the disclosure and are not intended to limit the disclosure.
FIG. 1 shows a fitted curve for HBV DNA inhibition rate of Embodiment 6.
FIG. 2 shows a fitted curve for HBsAg inhibition rate of Embodiment 6.
FIG. 3 shows a fitted curve for HBeAg inhibition of Embodiment 6.
FIG. 4 shows a fitted curve for HBV RNA inhibition rate of Embodiment 6.
FIG. 5 shows a fitted curve for HBV DNA inhibition rate of Embodiment 7.
FIG. 6 shows a fitted curve for HBsAg inhibition rate of Embodiment 7.
FIG. 7 shows a fitted curve for HBeAg inhibition of Embodiment 7.
FIG. 8 shows a fitted curve for HBV RNA inhibition rate of Embodiment 7.
FIG. 9 shows a fitted curve for HBV DNA inhibition rate of Embodiment 8.
FIG. 10 shows a fitted curve for HBsAg inhibition rate of Embodiment 8.
FIG. 11 shows a fitted curve for HBeAg inhibition of Embodiment 8.
FIG. 12 shows a fitted curve for HBV RNA inhibition rate of Embodiment 8.
FIG. 13 shows an HBV DNA content in plasma of C57BL/6-HBV transgenic model mice in Embodiment 9.
FIG. 14 shows an HBsAg content in plasma of C57BL/6-HBV transgenic model mice in Embodiment 9.
FIG. 15 shows an HBeAg content in plasma of C57BL/6-HBV transgenic model mice in Embodiment 9.

### DETAILED DESCRIPTION

Technical solutions in embodiments of the disclosure will be clearly and completely described below with reference to the accompanying drawings. Obviously, the described embodiments are only a part of the embodiments of the disclosure, and not all of the embodiments. Based on the embodiments in the disclosure, all other embodiments obtained by those skilled in the art without creative effort are within the scope of protection of the disclosure.

Unless otherwise indicated, the following terms have the meanings described below. Any undefined term has its generally accepted meaning in the field.

An "oligonucleotide compound" is a linear polymer compound consisting of 13-24 linked nucleosides, covalently linked by phosphate groups such as phosphate, phosphorothioate, aminoalkyl phosphate, and alkyl phosphate.

When the oligonucleotide compound is represented by nucleic acid base sequences such as "CGCTGATTTG", unless otherwise indicated, the sequence should be understood to be in a 5'→3' order from left to right, and "A" represents adenine, "G" represents guanine, "C" represents cytosine, "T" represents thymine (T), and "U" represents uracil (U).

A "nucleoside" is a compound consisting of a ribose (or deoxyribose) and a nucleobase (purine or pyrimidine) linked by a glycosidic bond, and is a basic structural unit of nucleic acids. Nucleosides may include natural nucleosides in 2'-deoxy and 2'-hydroxy forms, and may also include modified nucleosides with modified base moieties and/or modified sugar moieties.

"Base" refers to standard DNA and RNA bases (uracil, thymine, adenine, guanine, and cytosine), and also includes modified bases, such as 5-methylcytosine.

"5-Methylcytosine" refers to cytosine modified with a methyl group linked to the 5-position. 5-Methylcytosine is a modified nucleic acid base.

"cLNA" and "carbocyclic nucleoside" refer to a carbocyclic nucleoside containing the structure of formula (I) herein.

"2'-O-methoxyethyl" (also known as 2'-MOE and 2'-O(CH₂)₂-OCH₃) refers to the O-methoxy-ethyl modification at the 2'-position of the furanose ring. Sugars modified with 2'-O-methoxyethyl are modified sugars.

"2'-MOE nucleoside" (also known as 2'-O-methoxyethyl nucleoside) refers to a nucleoside containing a 2'-MOE modified sugar moiety.

"2'-substituted nucleosides" refers to nucleosides containing substituents other than H or OH at the 2'-position of the furanyl ring.

"Phosphothioester linking" or "PS linking" refers to a modified phosphate linking in which one of the non-bridging oxygen atoms is replaced by a sulfur atom.

"Gapmer" refers to a chimeric antisense compound having an internal region constituted by multiple nucleosides supporting ribonuclease H cleavage and situated between external regions having one or more nucleosides, where the nucleosides constituting the internal region are chemically different from those constituting the external region. The internal region may be referred to as the "intergenic region" and the external region as the "flanking region".

"Lipophilic group" refers to a group derived from any lipid-soluble molecule, such as fats, oils, waxes, terpenes, sterols, lipid-soluble vitamins (e.g., A, D, E, and K), monoglycerides, diglycerides, triglycerides, fatty acids, hopanoids, and phospholipids.

"Linker" refers to the organic part that connects two parts of a compound.

"Pharmaceutically acceptable salt" refers to a physiologically and pharmaceutically acceptable salt of a compound that retains the desired biological activity of the parent compound without imparting undesired toxic effects thereto.

"Pharmaceutically acceptable carrier or diluent" refers to a medium or diluent that does not interfere with the structure or function of oligonucleotides, an adjunct other than oligonucleotides administered to animals or humans. Some such carriers enable the formulation of pharmaceutical compositions into oral preparations such as tablets, capsules, liquids, suspensions, etc., and some such carriers enable the formulation of pharmaceutical compositions into injectable or infusion preparations. For example, a pharmaceutically acceptable carrier can be a sterile aqueous solution or physiological saline.

"Agent" or "therapeutic agent" means an active substance that provides therapeutic benefit when administered to an animal or human. "First agent" means a therapeutic compound described herein. For example, the first agent may be an antisense oligonucleotide targeting HBV and/or HDV. "Second agent" means at least one of other pharmaceutically acceptable active ingredients, such as a second therapeutic compound (e.g., a second antisense oligonucleotide targeting HBV and/or HDV) and non-HBV and/or HDV therapeutic compounds.

"Prevention" means, for example, the complete or near-complete prevention of the occurrence of a disease or condition (e.g., infection, ischemia, or reperfusion injury) when a patient or subject is susceptible to or at risk of the disease or condition; and prevention may also include inhibition, i.e., preventing the development of the condition.

"Treatment" means: 1) inhibiting the disease; for example, inhibiting the disease, condition, or symptom in an individual experiencing or exhibiting the pathology or symptom of the disease (i.e., preventing further development of the pathology and/or symptom); or 2) improving the disease; for example, ameliorating the disease, condition, or symptom in an individual experiencing or exhibiting the pathology or symptom (i.e., reversing the pathology and/or symptom).

"Animal" means human or non-human animal, including but not limited to mice, rats, rabbits, dogs, cats, pigs, and non-human primates, including but not limited to monkeys and chimpanzees.

"Administration" means providing an agent to an animal or individual, including but not limited to administration by a medical professional and self-administration.

"Simultaneous administration" refers to the combined administration of two agents in any manner in which their pharmacological effects are simultaneously manifested in the patient's body. Simultaneous administration does not require that the two agents be administered in a single pharmaceutical composition, in the same dosage form, or via the same route of administration. The effects of the two agents themselves do not need to manifest simultaneously. These effects only need to overlap for a period of time without being prolonged together.

"HBV" means mammalian hepatitis B virus, including human hepatitis B virus. The terminology encompasses hepatitis B virus, particularly the geographic genotype of human hepatitis B virus, and variant strains of the geographic genotype of hepatitis B virus.

"HBV antigen" means any hepatitis B virus antigen or protein, including core proteins such as "hepatitis B core antigen" or "HBcAg" or "HBcAG", "hepatitis B e antigen" or "HBeAg" or "HBeAG", and envelope proteins such as "HBV surface antigen" or "HBsAg" or "HBsAG".

"HBV RNA" means any messenger RNA expressed by hepatitis B virus.

"HBV nucleic acid" or "HBV DNA" means any nucleic acid encoding HBV. For example, in some embodiments, HBV nucleic acid includes, but is not limited to, any viral DNA sequence encoding the HBV genome or a portion thereof, any RNA sequence transcribed from viral DNA, including any RNA sequence encoding HBV protein.

"HBV protein" means any protein secreted by hepatitis B virus, which encompasses various HBV antigens, including core proteins such as "Hepatitis E antigen", "HBeAg", or "HBeAG", and envelope proteins such as "HBV surface antigen", "HBsAg", or "HBsAG."

"Hepatitis B-related disease or symptoms" or "HBV-related disease or symptoms" means any disease, biological symptom, medical symptom, or event caused by, related to, or attributable to hepatitis B infection, exposure, or exacerbation. The term "hepatitis B-related symptoms" includes chronic HBV infection, inflammation, fibrosis, cirrhosis, liver cancer, serological hepatitis, jaundice, liver inflammation, liver fibrosis, cirrhosis, liver failure, diffuse hepatocellular inflammatory disease, hemophagocytic syndrome, HBV viremia, liver transplantation related to liver disease, and symptoms including any one or all of the following when combined with a positive test for the presence of hepatitis B virus, hepatitis B virus antigen, or a positive test for the presence of hepatitis B virus antigen-specific antibodies: influenza-like symptoms, weakness, pain, headache, fever, loss of appetite, diarrhea, nausea and vomiting, pain in the liver area, clay-colored or gray stools, generalized itching, and dark urine.

"HDV (hepatitis D virus)" refers to hepatitis D virus, i.e., a defective virus.

Terms such as "induction", "inhibition", "enhancement", "elevation", "increase", "decrease", or similar terms generally indicate a quantitative difference between two states. These terms may refer to a statistically significant difference between two states. For example, "effective inhibition of HBV activity or expression" means that the level of HBV activity or expression in the treated sample differs quantitatively from the level of HBV activity or expression in untreated cells, and may be statistically significant. These terms apply to, for example, expression levels and activity levels. "Inhibition of HBV" means a reduction in the level or expression of HBV RNA, DNA, and/or protein. In some embodiments, HBV is inhibited in the presence of an antisense compound targeting HBV (including antisense oligonucleotides targeting HBV) compared to the expression levels of HBV RNA, HBV DNA, and/or protein in the absence of an HBV antisense compound (such as an antisense oligonucleotide). In some embodiments, HBV RNA levels are reduced. In some embodiments, HBV DNA levels are reduced. In some embodiments, HBV protein levels are reduced. In some embodiments, HBV antigen levels are reduced. In some embodiments, HBV s antigen (HBsAg) levels are reduced. In some embodiments, HBV e antigen (HBeAg) levels are reduced. The reduction may occur in a time-dependent manner and/or a dose-dependent manner.

In the chemical formula provided in the disclosure, DMTr is 4,4'-dimethoxytriphenylmethyl, Bn is benzyl, Ms is methanesulfonyl, Bz is benzoyl, Me is methyl, Ac is acetate, and (S) and (R) represent the S configuration and R configuration, respectively.

The disclosure provides an oligonucleotide compound. The oligonucleotide compound includes at least one carbocyclic nucleoside of formula (I), and a nucleic acid base sequence of each of the at least one oligonucleotide compound is as shown in sequence identity 1 (SEQ ID NO.1), 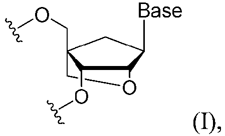 where Base is a heterocyclic base, 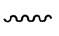 indicates the linking site.

The oligonucleotide compound of the disclosure can target HBV DNA transcripts. Simultaneously, the carbocyclic nucleoside can enhance resistance to nucleases and thermal stability of the overall structure, increase the base pairing affinity and specificity of the oligonucleotide compound, promote the binding of the oligonucleotide compound with complementary mRNA, and enhance the efficacy and safety of the oligonucleotide compound. This is beneficial for reducing serum HBeAg, HBsAg, and HBV DNA levels, thereby preventing and treating HBV-related diseases or symptoms. HDV infection requires the presence of HBV. HDV relies on HBV-derived HBsAg for packaging and viral transmission in the liver. HBV and HDV both utilize HBsAg. The oligonucleotide compound provided in the disclosure can also effectively reduce HBV DNA transcripts, reduce HBsAg production, and exert antiviral effects against HDV, thereby preventing and treating HDV-related diseases or symptoms, providing a new treatment method for hepatitis B and hepatitis D.

The oligonucleotide compound of the disclosure may include at least one carbocyclic nucleoside of formula (I). For example, the oligonucleotide compound contains one carbocyclic nucleoside of formula (I); or, the oligonucleotide compound has multiple (e.g., two, three, four, etc.) carbocyclic nucleosides of formula (I).

It can be understood that the heterocyclic base in formula (I) is any one base in the nucleic acid base sequence of the oligonucleotide compound. In one embodiment of the disclosure, the heterocyclic base may include at least one member selected from the group consisting of adenine, guanine, cytosine, 5-methylcytosine, thymine, and uracil.

In one embodiment of the disclosure, the oligonucleotide compound consists of a 5'-flanking segment, an intergenic segment, and a 3'-flanking segment. A base of the 5'-flanking segment includes GCAGA, a base of the intergenic segment includes GGTGAAGCGA, and a base of the 3'-flanking segment includes AGTGC. In some embodiments, the nucleotides in the 5'-flanking segment and 3'-flanking segment are ribonucleotides, and the nucleotide in the intergenic segment are deoxyribonucleotide. In some embodiments, nucleosides in the 5'-flanking segment and 3'-flanking segment are modified with 2'-O-methoxyethyl. It can be understood that the nucleosides in the 5'-flanking segment and 3'-flanking segment may be partially or completely modified with 2'-O-methoxyethyl. In some embodiments, adjacent nucleosides in the oligonucleotide compound are linked by a phosphorothioate. In some embodiments, all cytosines in the oligonucleotide compound are 5-methylcytosine.

In one embodiment of the disclosure, the 5' flanking segment and/or the 3' flanking segment contain the carbocyclic nucleoside, which enhances the structural stability and targeting of the oligonucleotide compound.

In one embodiment of the disclosure, the oligonucleotide compound further includes a lipophilic group. The lipophilic groups can improve the delivery ability of the oligonucleotide compound to liver cells, increase its cell permeability, enhance intracellular medicament delivery efficiency, and improve the distribution characteristics of intracellular oligonucleotide compounds, thereby enabling faster, more direct, and more efficient targeting of HBV DNA transcripts. Simultaneously, the oligonucleotide compound containing the carbocyclic nucleoside and the lipophilic group has advantages such as good stability, strong in vitro and in vivo anti-HBV activity, and long duration of action. The oligonucleotide compound can also act on HDV, which is beneficial for the prevention and improvement of HBV and HDV-related diseases or symptoms, providing a new treatment method for hepatitis B and hepatitis D.

In one embodiment of the disclosure, the lipophilic group is located at least one of the 5' and 3' ends of the oligonucleotide compound. That is, the lipophilic group may be located only at the 5' end of the oligonucleotide compound, or only at the 3' end of the oligonucleotide compound, or at both the 5' and 3' ends of the oligonucleotide compound. In other words, the lipophilic group is located at least one of the 5' and 3' ends of the nucleic acid base sequence of the oligonucleotide compound, which can also be understood as the lipophilic group being located at one or both ends of the molecular chain of the oligonucleotide compound. The conjugated lipophilic group can enhance the penetration and delivery of the oligonucleotide compound without affecting the targeting effect of the oligonucleotide compound.

In one embodiment of the disclosure, the lipophilic group is derived from a lipophilic compound having 4-32 carbon atoms, thereby enhancing the cell penetration ability of the oligonucleotide compound. Specifically, the number of carbon atoms in the lipophilic compound (i.e., the number of carbon atoms in the lipophilic group) may be, but is not limited to, 4, 5, 6, 7, 8, 9, 10, 11, 12, 13, 14, 15, 16, 17, 18, 19, 20, 21, 22, 23, 24, 25, 26, 27, 28, 29, 30, 31, or 32. In some embodiments, the lipophilic compound include at least one member selected from the group consisting of alkane, an alkanoyl compound, a carboxyl compound, a retinyl compound, cholestene, cholesterol, dihydrotestosterone, hexadecylglycerol (such as 1,3-di-O-(hexadecyl)glycerol), menthol, borneol, a dimethoxytriphenylmethyl compound, a geranyloxyhexyl compound, and phenoxazine. Exemplarily, the alkane may be C₄-C₃₂ alkane, i.e., the lipophilic group is a C₄-C₃₂ alkyl group. Exemplarily, the alkanoyl compound may be a C₄-C₃₂ alkanoyl compound, i.e., the lipophilic group is a C₄-C₃₂ alkanoyl group. Exemplarily, the carboxyl compound may include at least one member selected from the group consisting of cholic acid, adamantaneacetic acid, 1-pyrenebutyric acid, O3-(oleoyl)lithocholic acid, O3-(oleoyl)cholenic acid, and bile acid.

In one embodiment of the disclosure, the oligonucleotide compound further includes a linker, the linker is linked to the lipophilic group, and the linker includes a biologically cleavable group. In some embodiments, the biologically cleavable group include at least one member selected from the group consisting of phosphate and phosphorothioate. In some embodiments, the linker is a biologically cleavable group. That is, the linker contains only a biologically cleavable group. In some embodiments, the linker may further contain a linking group, that is, the linker includes a biologically cleavable group and a linking group, and the linking group links the biologically cleavable group and the lipophilic group. The linking group may be covalently coupled to the lipophilic group. In some embodiments, the linking group includes at least one member selected from the group consisting of groups as shown in formulas (II-1) to (II-5),

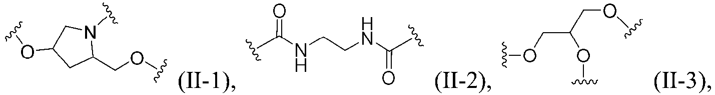

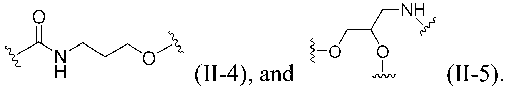

In one embodiment of the disclosure, a combination of the linker and the lipophilic group includes at least one member selected from the group consisting of groups as show in formulas (III-1) to (III-4), 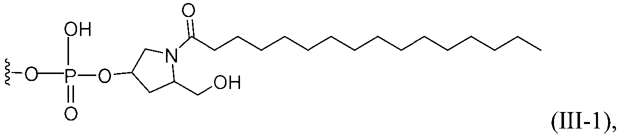 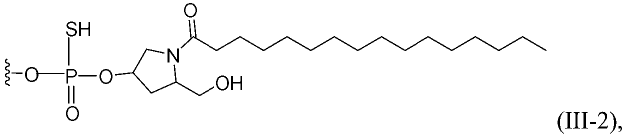 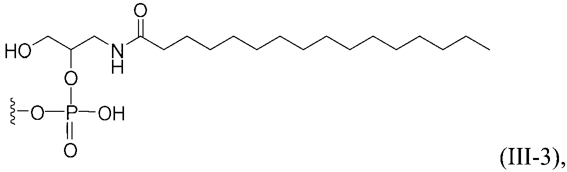 and

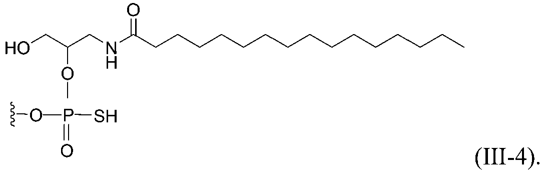

The disclosure also provides a medicament including the oligonucleotide compound in any of the above embodiments or a pharmaceutically acceptable salt thereof. This medicament has excellent structural stability, specific targeting, and biological activity, and can reduce expression levels of HBV RNA, HBV DNA, and related proteins, which is beneficial for the prevention and treatment of HBV and HDV-related diseases or symptoms.

In one embodiment of the disclosure, the pharmaceutically acceptable salt of the oligonucleotide compound can be at least one of sodium or potassium salts.

In one embodiment of the disclosure, the medicament may further include a pharmaceutically acceptable carrier or diluent, which facilitates medicament administration.

In one embodiment of the disclosure, the oligonucleotide compound or the pharmaceutically acceptable salt thereof constitutes the medicament as a single active ingredient or together with another pharmaceutically acceptable active ingredient. That is, the oligonucleotide compound or the pharmaceutically acceptable salt thereof is the sole active ingredient in the medicament, or the oligonucleotide compound or the pharmaceutically acceptable salt thereof and another pharmaceutically acceptable active ingredient constitute the active ingredients in the medicament. The another pharmaceutically acceptable active ingredient constitutes a second agent, which may be, but is not limited to, selected from the group consisting of an anti-HBV agent, a chemotherapeutic agent, an antibiotic, an analgesic, an antiinflammatory agent, an antifungal agent, an antiparasitic agent, an antinausea agent, an antidiarrheal agent, an immunomodulator, and other antiviral agent. The second agent may be used to treat hepatitis B, cancer, bacteria, pain, inflammation, fungal infections and/or parasitic infections, alcoholism, substance abuse, diabetes, or may be used to treat other viral infections such as human immunodeficiency virus (HIV), hepatitis C virus (HCV), etc. In some embodiments, the anti-HBV agent may be selected from, but is not limited to, interferons (including but not limited to, interferon α2b, interferon α2a, interferon α-1a, pegylated interferon α2a, pegylated interferon α2b), nucleoside (acid) analogs (including but not limited to entecavir; tenofovir disoproxil fumarate; tenofovir alafenamide fumarate; tenofovir amibufenamide), other anti-HBV small nucleic acid medicaments, Toll-like receptor (TLR) agonists, capsid inhibitors, HBV therapeutic vaccines, HBV preventive vaccines, neutralizing antibodies, HBV monoclonal or polyclonal antibody therapeutics, etc. In some embodiments, other anti-HBV small nucleic acid medicaments may be selected, but are not limited to, antisense oligonucleotides (ASO), small interfering RNA (siRNA), aptamer RNA, microRNA (miRNA), circular RNA (circRNA), small activating RNA (saRNA), CpG oligonucleotides, CRISPR-Cas nucleic acids, ribozymes, transfer RNA (tRNA), antibody-nucleic acid conjugates (ARC), nucleic acid aptamermedicament conjugates (ADC), etc.

The disclosure also provides the use of the oligonucleotide compound or the pharmaceutically acceptable salt thereof in any of the above embodiments, or the medicament in any of the above embodiments, in preparation of a medicament for prevention and/or treatment of HBV and/or HDV-related diseases or symptoms, which is beneficial for the prevention and treatment of HBV and/or HDV-related diseases or symptoms.

In one embodiment of the disclosure, the medicament may further include a second agent. The oligonucleotide compound or the pharmaceutically acceptable salt thereof and the second agent may be administered simultaneously in a single formulation or separately in different formulations, and the administration of the oligonucleotide compound or the pharmaceutically acceptable salt thereof and the second agent may be performed in parallel or sequentially.

The disclosure also provides a method of prevention and/or treatment, including administering to a subject the oligonucleotide compound or the pharmaceutically acceptable salt thereof in any of the above embodiments, or the medicament in any of the above embodiments, thereby preventing and/or treating HBV and/or HDV-related diseases or symptoms.

In one embodiment of the disclosure, the medicament includes a second agent, and the treatment method further includes administering to a subject the oligonucleotide compound or the pharmaceutically acceptable salt thereof as described in any of the above embodiments and the second agent. The oligonucleotide compound or the pharmaceutically acceptable salt thereof and the second agent may be administered simultaneously or separately.

The oligonucleotide compound provided in the disclosure exhibits high HBV inhibitory activity. In one embodiment of the disclosure, the oligonucleotide compound may include at least one of compounds 1 to 12 shown in Table 1.

**Table 1 Oligonucleotide Compounds**

| Compounds | Sequence (5'-3') |
|---|---|
| Positive Control | GCAGAggtgaagcgaAGTGC |
| Compound 1 | GC^{cLNA}AGAggtgaagcgaA^{cLNA}GT^{cLNA}GC |
| Compound 2 | GC^{cLNA}AGAggtgaagcga^{cLNA}AG^{cLNA}TGC |
| Compound 3 | ^{cLNA}GCAGAggtgaagcgaA^{cLNA}GT^{cLNA}GC |
| Compound 4 | Xₛ^{cLNA}GCAGAggtgaagcgaA^{cLNA}GT^{cLNA}GC |
| Compound 5 | ^{cLNA}GCAGAggtgaagcga^{cLNA}AGT^{cLNA}GC |
| Compound 6 | ^{cLNA}GCAGAggtgaagcgaAG^{cLNA}T^{cLNA}GC |
| Compound 7 | GC^{cLNA}A^{cLNA}GAggtgaagcgaA^{cLNA}GT^{cLNA}GC |
| Compound 8 | ^{cLNA}GC^{cLNA}AGAggtgaagcgaAG^{cLNA}T^{cLNA}GC |
| Compound 9 | GCAGAggtgaagcga^{cLNA}AGT^{cLNA}GC |
| Compound 10 | GCAGAggtgaagcgaA^{cLNA}GT^{cLNA}GC |
| Compound 11 | XₒGCAGAggtgaagcgaA^{cLNA}GT^{cLNA}GC |
| Compound 12 | XₒGCAGAggtgaagcga^{cLNA}AGT^{cLNA}GC |

In this structure, each nucleoside is linked by a phosphorothioate (PS) bond, and each cytosine is 5-methylcytosine, lowercase "a, g, c, t" represent deoxyribonucleosides, and uppercase "A, G, C, T" represent 2'-MOE-modified nucleosides; "^{cLNA}A" represents a carbocyclic nucleoside with base A, "^{cLNA}G" represents a carbocyclic nucleoside with base G, and "^{cLNA}T" represents a carbocyclic nucleoside with base T; "Xₒ" represents the following structure: 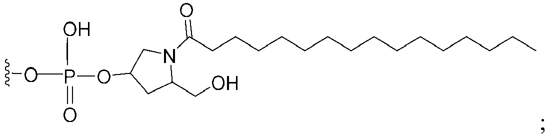 and "Xₛ" represents the following structure:

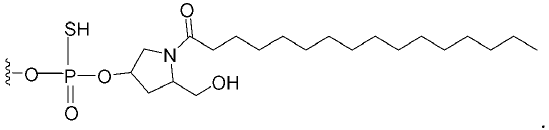

The effects of the oligonucleotide compound provided in the disclosure will be illustrated in more detail by the following examples, but the following examples are only used to illustrate the compound described herein and are not intended to limit the disclosure.

Embodiment 1: Synthesis of ^{cLNA}G phosphorous amide monomer (compound 1-1-7).

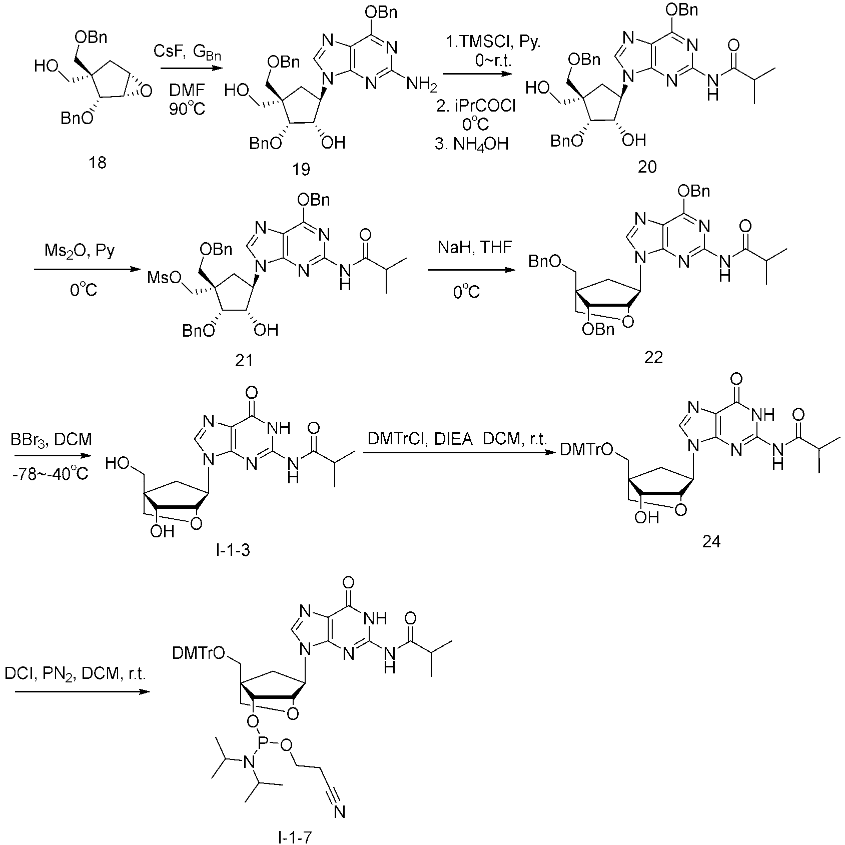

### (1) Synthesis of Compound 19

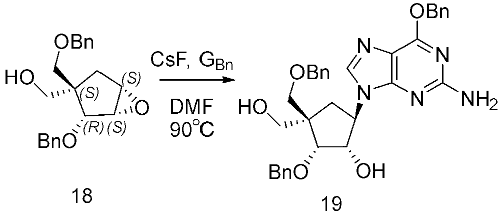

Under a nitrogen atmosphere, compound 18 (24 g, 0.07 mol), cesium fluoride (31.9 g, 0.21 mol), and O-6-benzylguanine (G_{Bn}, 33.8 g, 0.14 mol) are suspended in 240 mL of dry N,N-dimethylformamide (DMF). The mixture is heated to 90 °C and reacted for 3 h. The reaction is monitored by liquid chromatography-mass spectrometry (LCMS) until completion. The mixture is cooled, water is added, and the mixture is extracted twice with ethyl acetate. The organic phases are combined, washed twice with saturated brine, dried over anhydrous sodium sulfate, filtered, and concentrated. The crude product is purified by column chromatography with petroleum ether: ethyl acetate (v:v) = 1:2~1:3 as the eluent, yielding 23.3 g of oily substance, with a yield of 56.8%. [M+1]⁺=582.2;1H NMR (400 MHz, Chloroform-d) δ 7.64 (s, 1H), 7.57 - 7.47 (m, 2H), 7.46 - 7.29 (m, 13H), 5.58 (s, 2H), 4.94 (d, J = 11.4 Hz, 1H), 4.88 (s, 2H), 4.70 (dd, J = 10.8, 3.0 Hz, 2H), 4.57 (dd, J = 8.4, 5.6 Hz, 1H), 4.52 (s, 2H), 4.15 (d, J = 5.6 Hz, 1H), 3.85 (d, J = 11.3 Hz, 1H), 3.66 (d, J = 11.3 Hz, 1H), 3.41 (s, 2H), 2.38 (dd, J = 13.3, 8.7 Hz, 1H), 2.09 - 1.96 (m, 2H).

### (2) Synthesis of Compound 20

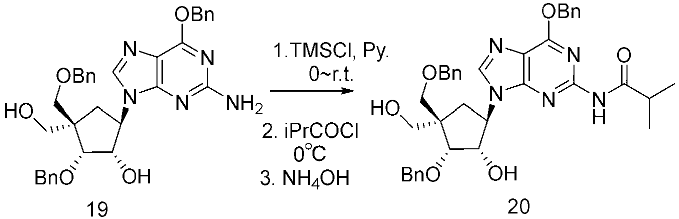

Under a nitrogen atmosphere, compound 19 (23.2 g, 0.04 mol) is dissolved in 230 mL of dry pyridine (Py.), cooled to 0 °C, and trimethylchlorosilane (TMSCl, 26.1 g, 0.24 mol) is slowly added dropwise. After the addition is complete, the mixture is stirred at room temperature (rt) for 2 h. The mixture is then cooled to 0 °C, and isobutyryl chloride (iPrCOCl, 4.7 g, 0.044 mol) is slowly added dropwise. After the addition is complete, the mixture is stirred for another 2 h. 30 mL of concentrated ammonia (NH₄OH) is added dropwise, and the mixture is stirred at room temperature for 1 h. The reaction is monitored by LCMS until complete. Water is added, and the mixture is extracted twice with ethyl acetate. The organic phases are combined, washed twice with saturated brine, dried over anhydrous sodium sulfate, filtered, and concentrated. The crude product is purified by column chromatography using petroleum ether:ethyl acetate (v:v) = 1:1~1:2, yielding 26.3 g of an oily liquid, with a yield of 97%. [M+1]⁺= 652.3.

### (3) Synthesis of Compound 21

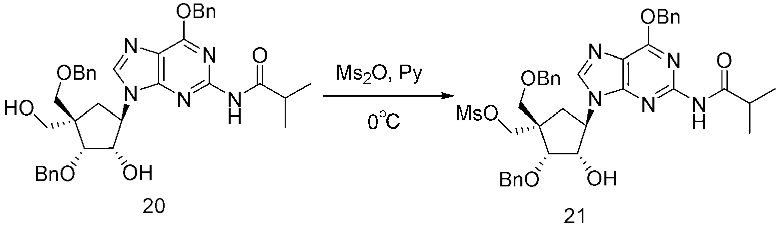

Under a nitrogen atmosphere, compound 20 (25.5 g, 0.039 mol) is dissolved in 200 mL of dry pyridine (Py). The solution is cooled to 0 °C, and a tetrahydrofuran solution of methanesulfonic anhydride (Ms₂O, 8.7 g, 0.051 mol) is slowly added dropwise. After the addition is complete, stirring is continued for 2 h. The reaction is monitored by LCMS until the product concentration reached its maximum. Water is added to quench the reaction, and the product is extracted twice with ethyl acetate. The combined organic phases are washed twice with saturated brine, dried over anhydrous sodium sulfate, filtered, and concentrated. The crude product is purified by column chromatography using petroleum ether:ethyl acetate (v:v) = 1:1, yielding 19.1 g of oil (56.8%). 4.5 g of the starting material is recovered. [M+1]⁺= 730.3.

### (4) Synthesis of Compound 22

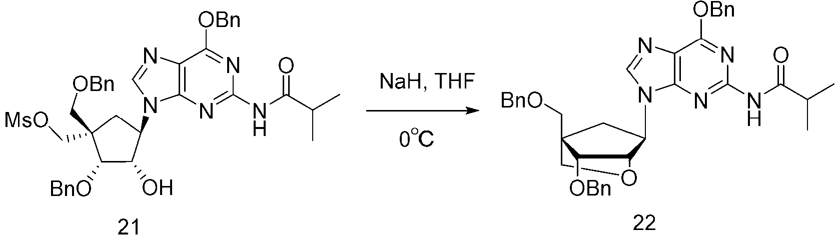

Under a nitrogen atmosphere, 60% sodium hydride (NaH, 3.5 g, 87.9 mmol) is carefully added to 210 mL of dry tetrahydrofuran (THF), and stirred for 10 min. The mixture is cooled to 0 °C, and a tetrahydrofuran solution of compound 21 (21.4 g, 29.3 mmol) is slowly added dropwise. After the addition is complete, stirring is continued for 2 h. The reaction is monitored by LCMS until complete. The reaction is quenched with acetic acid, and the mixture is extracted twice with ethyl acetate after adding saturated sodium bicarbonate solution. The organic phases are combined, washed successively with saturated brine, dried over anhydrous sodium sulfate, filtered, and concentrated. The crude product is purified by column chromatography with petroleum ether:ethyl acetate (v:v) = 1:1 as the eluent, yielding 15.8 g of oily product, 85% yield. [M+1]⁺= 634.3.

### (5) Synthesis of compound I-1-3

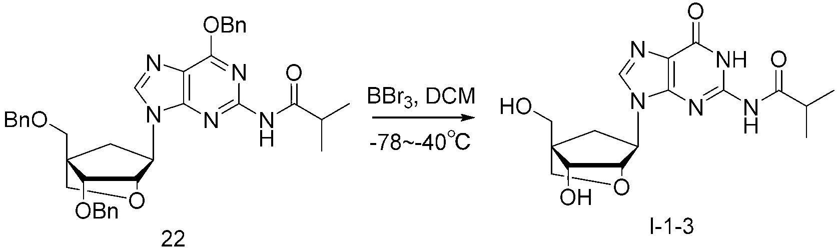

Under a nitrogen atmosphere, compound 22 (5 g, 7.9 mmol) is dissolved in 50 mL of dichloromethane (DCM), cooled to -78 °C, and 71 mL of a 1 M boron tribromide (BBr₃) solution in dichloromethane is slowly added dropwise. After the addition is complete, the temperature is slowly raised to -40 °C and stirred for 2 h. The reaction is monitored by LCMS until completion, quenched dropwise with methanol, and then heated to room temperature and concentrated. The crude product is purified by column chromatography with dichloromethane:methanol (v:v) = 10:1 as the eluent, yielding 2.5 g of white solid, with a yield of 87.2%. [M+1]⁺= 364.1;1H NMR (400 MHz, DMSO-d6) δ 8.14 (s, 1H), 7.36 - 7.21 (m, 1H), 5.17 (d, J = 4.2 Hz, 1H), 4.59 (t, J = 5.2 Hz, 1H), 4.44 (dd, J = 9.7, 5.4 Hz, 1H), 4.03 (d, J = 4.4 Hz, 1H), 3.96 (s, 1H), 3.78 - 3.68 (m, 2H), 2.79 (p, J = 6.8 Hz, 1H), 2.23 (ddd, J = 26.1, 14.8, 10.2 Hz, 2H), 1.99 (dd, J = 10.2, 6.6 Hz, 1H), 1.12 (d, J = 6.8 Hz, 5H).

### (6) Synthesis of Compound 24

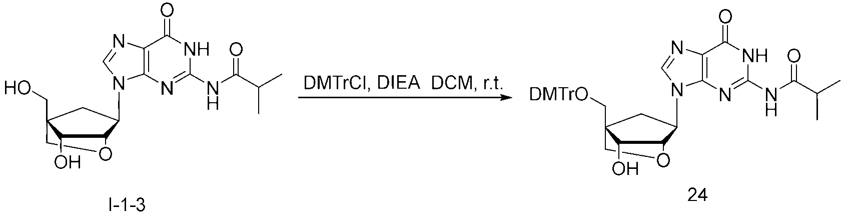

Under a nitrogen atmosphere, compound I-1-3 (3.1 g, 9.1 mmol) and 4,4'-bismethoxytriphenylmethyl chloride (DMTrCl, 6.8 g, 20.2 mmol) are dissolved in 30 mL of dry dichloromethane (DCM). The solution is cooled to 0 °C, and N,N-diisopropylethylamine (DIEA, 5.89 g, 45.5 mmol) is slowly added dropwise. After the addition is complete, the solution is heated to room temperature (rt) and stirred for 2 h. The reaction is monitored by LCMS until completion. The solution is concentrated, and the crude product is purified by column chromatography with dichloromethane:methanol:triethylamine (v:v:v) = 30:1:0.1% as the eluent, yielding 4.6 g of a pale yellow solid, with a yield of 76%. [M+1]⁺=666.3;1H NMR (400 MHz, Chloroform-d) δ 12.10 (s, 1H), 9.40 (s, 1H), 7.75 (s, 1H), 7.46 - 7.40 (m, 2H), 7.34 - 7.25 (m, 5H), 7.23 - 7.17 (m, 2H), 6.83 (ddd, J = 9.0, 4.2, 2.0 Hz, 4H), 4.66 (s, 1H), 4.48 (t, J = 7.6 Hz, 1H), 4.27 (s, 1H), 3.95 (d, J = 7.1 Hz, 1H), 3.82 (s, 1H), 3.78 (s, 6H), 3.68 (d, J = 7.0 Hz, 1H), 3.46 (d, J = 9.6 Hz, 1H), 3.27 (d, J = 9.6 Hz, 1H), 2.53 (p, J = 6.9 Hz, 1H), 2.32 (d, J = 7.6 Hz, 2H), 1.19 (dd, J = 9.7, 6.8 Hz, 6H).

### (7) Synthesis of compound I-1-7

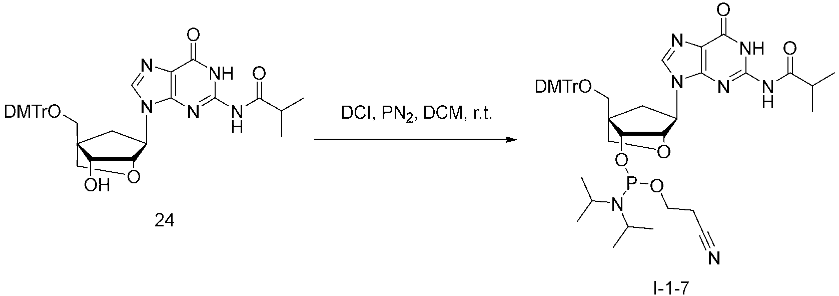

Under a nitrogen atmosphere, compound 24 (1.0 g, 1.5 mmol) and 4,5-dicyanimidazole (DCl, 443 mg, 3.75 mmol) are dissolved in 10 mL of dry dichloromethane (DCM). Bis(diisopropylamino)(2-cyanoethoxy)phosphine (PN₂, 1.1 g, 3.75 mmol) is added dropwise at room temperature (rt), and stirring is continued for 2 h. After the reaction is monitored by LCMS, dichloromethane and 0.1 mL of triethylamine are added. The organic phase is washed sequentially with saturated brine, dried over anhydrous sodium sulfate, filtered, and concentrated. The crude product is separated using high presser liquid chromatography (HPLC) on a C18 column with a mobile phase of acetonitrile (containing 0.0035% diisopropylamine):water (containing 0.0035% diisopropylamine) (v:v) = 7:3, yielding 660 mg of a white solid, with a yield of 51%. [M+1]⁺=866.4;³¹PNMR:147.27,147.17.

### Embodiment 2: Synthesis of^{cLNA}A phosphorus amide monomer (compound I-1-8).

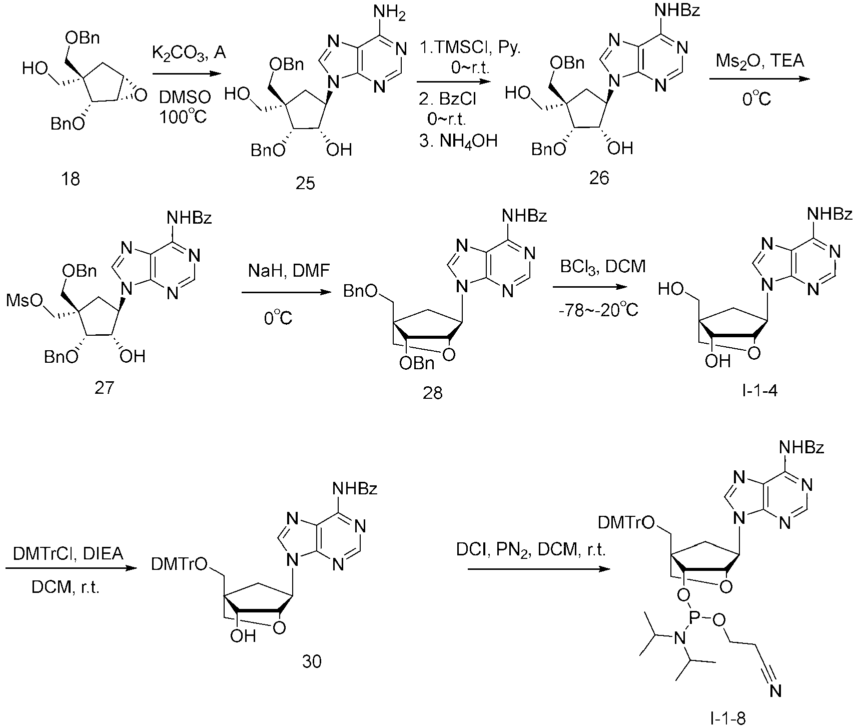

### (1) Synthesis of Compound 25

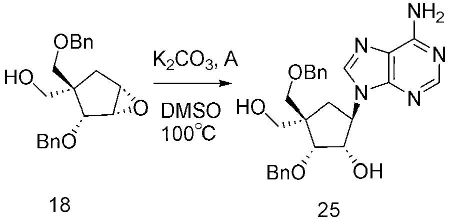

Under a nitrogen atmosphere, compound 18 (26 g, 0.076 mol), adenine (A, 20.5 g, 0.15 mol), and potassium carbonate (K₂CO₃, 31.5 g, 0.23) are suspended in 260 mL of dry dimethyl sulfoxide (DMSO) and heated to 120 °C for 4 h. After the reaction is completed by LCMS, the temperature is loared, water is added, and the mixture is extracted twice with ethyl acetate. The organic phases are combined, washed twice with saturated brine, dried over anhydrous sodium sulfate, filtered, and concentrated. The crude product is purified by column chromatography with dichloromethane:methanol (v:v) = 20:1 as the eluent, yielding 29.2 g of white solid, with a yield of 80.4%. [M+1]⁺=476.2;1H NMR (400 MHz, DMSO-d6) δ 8.14 (s, 1H), 8.10 (s, 1H), 7.43 - 7.24 (m, 10H), 7.16 (s, 2H), 5.19 (d, J = 6.2 Hz, 1H), 4.84 (d, J = 11.8 Hz, 1H), 4.81 - 4.71 (m, 2H), 4.61 - 4.53 (m, 3H), 4.50 (t, J = 4.9 Hz, 1H), 3.90 (d, J = 4.3 Hz, 1H), 3.65 - 3.56 (m, 2H), 3.54 (d, J = 4.6 2.10 (dd, J = 13.5, 8.6 Hz, 1H), 1.82 (dd, J = 13.4, 9.2 Hz, 1H).

### (2) Synthesis of Compound 26

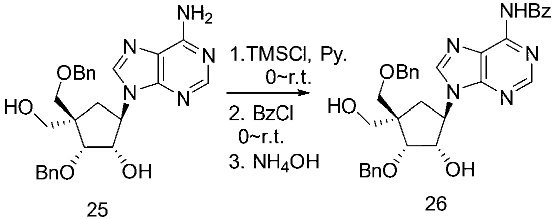

Under a nitrogen atmosphere, compound 25 (22 g, 0.046 mol) is dissolved in 200 mL of dry pyridine (Py.), cooled to 0 °C, and trimethylchlorosilane (TMSCl, 25 g, 0.231 mol) is slowly added dropwise. After the addition is complete, the mixture is heated to room temperature and stirred for 2 h. Benzoyl chloride (BzCl, 13.5 g, 0.097 mol) is slowly added dropwise. After the addition is complete, the mixture is heated to room temperature and stirred for 2 h. The reaction is monitored by LCMS until complete. The mixture is cooled to 0 °C, and 35 mL of concentrated ammonia (NH₄OH) is added. The mixture is slowly heated to room temperature and stirred for 1 h. The reaction is monitored by LCMS until complete. Water is added, and the mixture is extracted twice with ethyl acetate. The organic phases are combined, washed twice with saturated brine, dried over anhydrous sodium sulfate, filtered, and concentrated. The crude product is purified by column chromatography using 100% ethyl acetate as the eluent, yielding 20 g, with a yield of 73.7%. [M+1]⁺= 560.3.

### (3) Synthesis of Compound 27

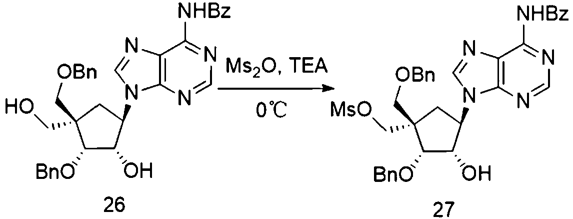

Under a nitrogen atmosphere, compound 26 (20 g, 0.034 mol) and methanesulfonic anhydride (Ms₂O, 7.2 g, 0.041 mol) are dissolved in 200 mL of dry tetrahydrofuran. The mixture is cooled to -10 °C, and triethylamine (TEA, 10.4 g, 0.103 mol) is slowly added dropwise. After the addition is complete, the mixture is heated to 0 °C and reacted for 2 h. The reaction is monitored by LCMS until the reactants are almost completely reacted. Saturated sodium bicarbonate aqueous solution is added, and the mixture is extracted twice with ethyl acetate. The organic phases are combined, washed successively with saturated brine, dried over anhydrous sodium sulfate, filtered, and concentrated. The crude product is purified by column chromatography with petroleum ether:ethyl acetate (v:v) = 1:1 as the eluent, yielding 17 g of white solid, with a yield of 65%. [M+1]⁺=658.2.

### (4) Synthesis of Compound 28

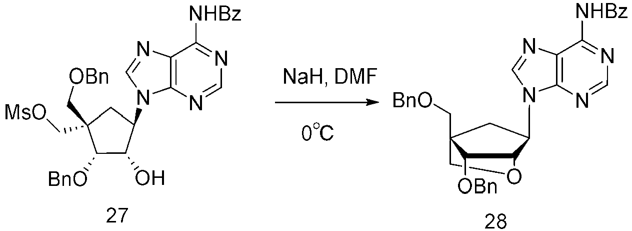

Under a nitrogen atmosphere, 60% sodium hydride (NaH, 1.87 g, 77.7 mmol) is carefully added to 170 mL of dry N,N-dimethylformamide (DMF), stirred for 10 min, cooled to 0 °C, and a solution of compound 27 (17 g, 25.9 mmol) in N,N-dimethylformamide is slowly added dropwise. After the addition is complete, stirring is continued for 2 h. The reaction is monitored by LCMS until completion. Saturated ammonium chloride solution is added, and the mixture is extracted twice with ethyl acetate. The organic phases are combined, washed successively with saturated brine, dried over anhydrous sodium sulfate, filtered, concentrated, and the crude product is purified by column chromatography with 100% ethyl acetate as the eluent, yielding 12.3 g of white solid, with a yield of 85%. [M+1]⁺= 562.2.

### (5) Synthesis of compound I-1-4

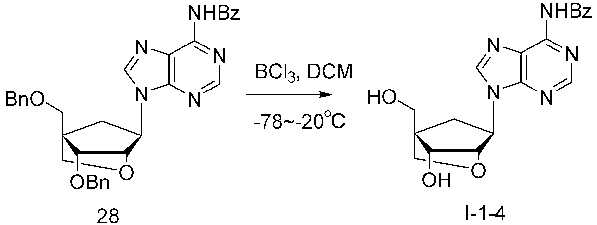

Under a nitrogen atmosphere, compound 28 (5.3 g, 10.7 mmol) is dissolved in 50 mL of dry dichloromethane (DCM) solution. The solution is cooled to -78 °C, and 64 mL of 1 M boron trichloride (BCl₃) solution in dichloromethane is slowly added dropwise. After the addition is complete, the solution is heated to -20 °C and stirred overnight. The reaction is monitored by LCMS until complete. The reaction is quenched with methanol, concentrated under reduced pressure, and then slurried with ethyl acetate. The mixture is filtered to give 3.4 g of a white solid, which is the hydrochloride salt of compound 1-1-4. [M+1]⁺=382.2;1H NMR (400 MHz, DMSO-d6) δ 11.16 (s, 1H), 8.71 (s, 1H), 8.55 (s, 1H), 8.11 - 8.02 (m, 2H), 7.71 - 7.63 (m, 1H), 7.56 (dd, J = 8.3, 7.0 Hz, 2H), 7.35 (d, J = 6.1 Hz, 5H), 7.32 - 7.23 (m, 5H), 4.79 - 4.70 (m, 1H), 4.66 - 4.59 (m, 1H), 4.55 (d, J = 4.7 Hz, 2H), 4.52 (d, J = 3.1 Hz, 1H), 4.29 (s, 1H), 3.85 (d, J = 6.7 Hz, 1H), 3.80 (d, J = 9.5 Hz, 1H), 3.73 (d, J = 6.5 Hz, 1H), 3.65 (d, J = 9.5 Hz, 1H), 2.44 (d, J = 9.1 Hz, 2H).

### (6) Synthesis of Compound 30

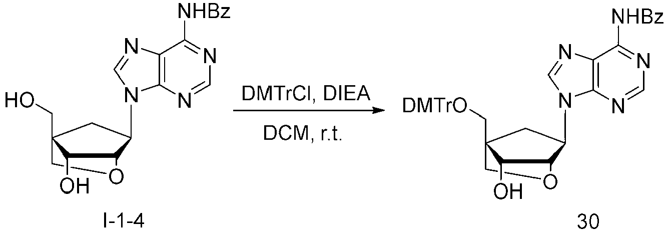

Under a nitrogen atmosphere, compound I-1-4 (1 g, 2.6 mmol) and 4,4'-bismethoxytriphenylmethyl chloride (DMTrCl, 1.9 g, 5.72 mmol) are dissolved in dry dichloromethane (DCM). The mixture is cooled to 0 °C, and N,N-diisopropylethylamine (DIEA, 1.3 g, 13 mmol) is slowly added dropwise. After the addition is complete, the mixture is heated to room temperature and stirred for 2 h. The reaction is monitored by LCMS until completion. The mixture is concentrated under reduced pressure, and the crude product is purified by column chromatography using dichloromethane:ethyl acetate:triethylamine (v:v:v) = 1:1:0.1% to give 1.3 g of a pale yellow solid, with a yield of 72.2%. [M+1]⁺=684.3;1H NMR (400 MHz, DMSO-d6) δ 11.16 (s, 1H), 8.76 (s, 1H), 8.62 (s, 1H), 8.05 (d, J = 7.7 Hz, 2H), 7.65 (t, J = 7.4 Hz, 1H), 7.56 (t, J = 7.6 Hz, 2H), 7.41 (d, J = 7.8 Hz, 2H), 7.33 (t, J = 7.6 Hz, 2H), 7.30 - 7.21 (m, 5H), 6.96 - 6.87 (m, 4H), 5.20 (d, J = 4.6 Hz, 1H), 4.75 (dd, J = 9.8, 5.5 Hz, 1H), 4.36 (d, J = 4.7 Hz, 1H), 4.11 (s, 1H), 3.81 (d, J = 6.5 Hz, 1H), 3.75 (s, 6H), 3.40 (d, J = 9.1 Hz, 1H), 3.21 - 3.16 (m, 2H), 2.57 - 2.52 (m, 1H), 2.46-2.39 (m, 1H).

### (7) Synthesis of compound I-1-8

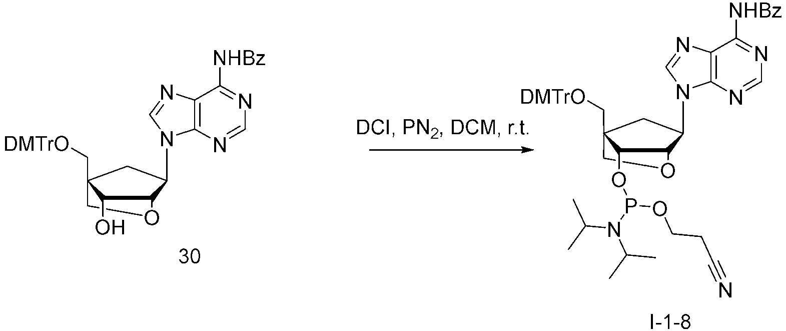

Under a nitrogen atmosphere, compound 30 (800 mg, 1.17 mmol) and 4,5-dicyanimidazole (DCl, 345 mg, 2.92 mmol) are dissolved in 10 mL of dry dichloromethane (DCM). Bis(diisopropylamino)(2-cyanoethoxy)phosphine (PN₂, 880 mg, 2.92 mmol) is added dropwise at room temperature (rt), and stirring is continued for 2 h. After the reaction is monitored by LCMS, dichloromethane and 0.1 mL of triethylamine are added. The organic phase is washed sequentially with saturated brine, dried over anhydrous sodium sulfate, filtered, and concentrated. The crude product is separated using high presser liquid chromatography (HPLC) with a C18 column and a mobile phase of acetonitrile (containing 0.0035% diisopropylamine):water (containing 0.0035% diisopropylamine) (v:v)=8:2, yielding 550 mg of white solid, 53% yield. [M+1]⁺=884.4;³¹PNMR:148.40,148.31.

### Embodiment 9 3: Synthesis of C₁₆phosphamide monomer (compound 33).

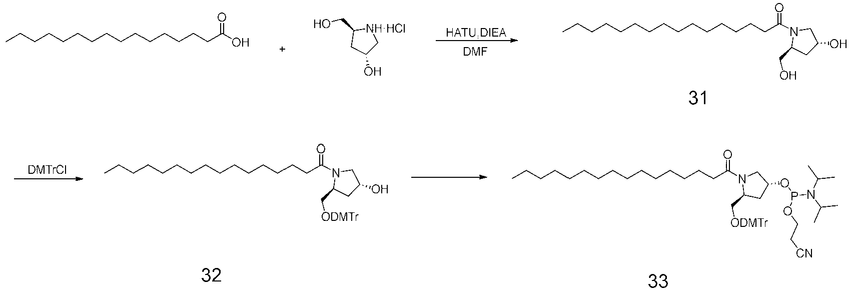

### (1) Synthesis of Compound 31

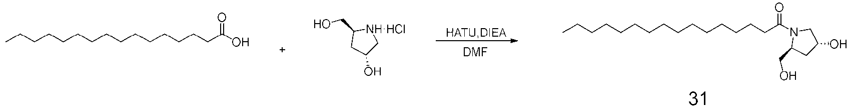

Under a nitrogen atmosphere, palmitic acid (20 g, 78.1 mmol), (3R,5S)-5-(hydroxymethyl)pyrrolidine-3-ol hydrochloride (12 g, 78.1 mmol), and HATU (C₁₀H₁₅OF₆N₆P, 44.5 g, 117.1 mmol) are suspended in 300 mL of dry N,N-dimethylformamide (DMF). The solution is cooled to 0 °C, and N,N-diisopropylethylamine (DIEA, 35.3 g, 273.4 mmol) is slowly added dropwise. After the addition is complete, the solution is stirred at room temperature for 3 h. The solution is then poured into 300 mL of water, and the pH is adjusted to approximately 8. After stirring for 1 h, the solution is filtered, washed with water, and the crude product is obtained. 140 mL of acetonitrile solution is added, and the solution is heated and stirred. The solution is then filtered, and the filter cake is dried to obtain 22 g of off-white solid, with a yield of 80%. [M+1]⁺=356;1H NMR (400 MHZ, DMSO-d6) δ4.28 (q, J= 4.5Hz,1H), 4.04-3.93(m,1H), 3.45(dq,J=14.7,5.0Hz,2H), 3.31-3.18(m,2H), 2.31-2.12(m,2H), 1.92(dt,J=13.0,5.7Hz,1H), 1.83-1.75(m,1H), 1.48(p,J=6.7Hz,2H), 1.24(m,24H), 0.93-0.81(m,3H).

### (2) Synthesis of Compound 32

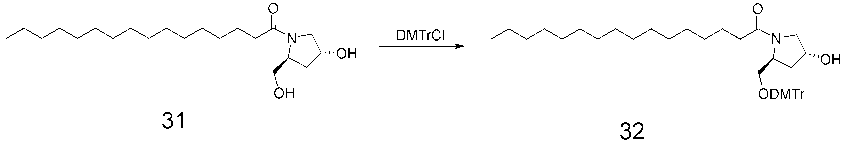

Under a nitrogen atmosphere, compound 31 (10 g, 28.1 mmol) and 4-dimethylaminopyridine (0.69 g, 5.6 mmol) are dissolved in 70 mL of pyridine. 4,4'-bismethoxytriphenylmethyl chloride (DMTrCl, 14.3 g, 42.1 mmol) is added in portions at 0 °C. After the addition is complete, the reaction is carried out at room temperature for 2 h. The reaction is monitored by TLC (dichloromethane:methanol (v:v) = 30:1). After the reaction is completed, ethyl acetate and water are added, and the mixture is separated. The aqueous phase is extracted once again with ethyl acetate. The organic phases are combined, washed with saturated brine, dried, filtered, and concentrated. The crude product is purified by column chromatography using 200-300 mesh neutral alumina. The eluent is dichloromethane:ethyl acetate (v:v) = 15:1~10:1. 1‰ triethylamine is added to the eluent to give 13.3 g of oil, with a yield of 72%. [M+1]⁺= 658.

### (3) Synthesis of Compound 33

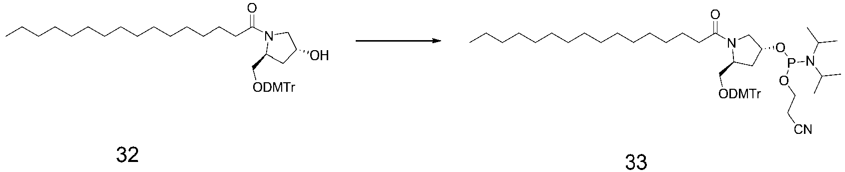

Under a nitrogen atmosphere, compound 32 (12 g, 18.3 mmol), N-methylimidazolium (1.5 g, 18.3 mmol), and tetrazolium (2.56 g, 36.6 mmol) are dissolved in 120 mL of dichloromethane. The mixture is cooled to 0 °C and bis(diisopropylamino)(2-cyanoethoxy)phosphine (16.5 g, 54.9 mmol) is added dropwise. After the addition is complete, the reaction is carried out at room temperature. After the reaction is monitored by LCMS, the organic phase is washed with saturated sodium bicarbonate aqueous solution and saturated brine, dried, and the crude product is separated by high presser liquid chromatography (HPLC) using a C18 column and acetonitrile (containing 0.0035% diisopropylamine) as the mobile phase, yielding 8.7 g of oily substance, with a yield of 56%. [M+1]⁺=858;³¹PNMR:148.1,147.8,147.6,147.3.

### Embodiment 4: Synthesis of^{cLNA}T phosphorus amide monomer (compound 1-1-5).

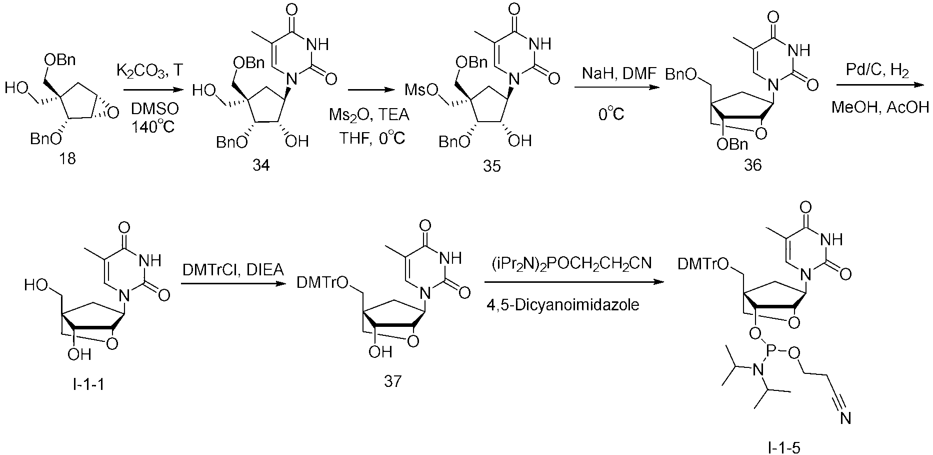

### (1) Synthesis of Compound 34

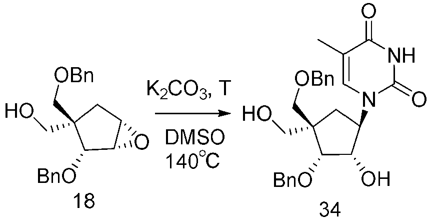

Under a nitrogen atmosphere, compound 18 (22 g, 0.065 mol), thymine (T, 32.8 g, 0.26 mol), and potassium carbonate (K₂CO₃, 26.9 g, 0.195 mol) are suspended in 220 mL of dry dimethyl sulfoxide (DMSO) and heated to 140 °C with stirring for 3 h. After the reaction is complete as detected by LCMS, the mixture is cooled to room temperature, water is added, and the mixture is extracted twice with ethyl acetate. The organic phases are combined, washed twice with saturated brine, dried over anhydrous sodium sulfate, filtered, and concentrated. The crude product is purified by column chromatography with dichloromethane:methanol (v:v) = 50:1~20:1 as the eluent, yielding 24.4 g of white solid, with a yield of 81%. [M+1]⁺=467.2;1H NMR (400 MHz, DMSO-d6) δ 11.19 (s, 1H), 7.55 - 7.16 (m, 11H), 5.09 (d, J = 6.5 Hz, 1H), 4.81 (d, J = 11.7 Hz, 1H), 4.71 (q, J = 9.7 Hz, 1H), 4.59 - 4.45 (m, 4H), 4.39 (dt, J = 9.7, 5.8 Hz, 1H), 3.80 (d, J = 4.9 Hz, 1H), 3.61 - 3.39 (m, 4H), 1.93 - 1.82 (m, 1H), 1.68 (d, J = 1.1 Hz, 3H), 1.35 (dd, J = 13.5, 10.1 Hz, 1H).

### (2) Synthesis of Compound 35

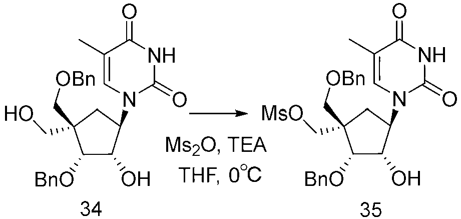

Under a nitrogen atmosphere, compound 34 (17.3 g, 0.037 mol) and triethylamine (TEA, 15 g, 0.148 mol) are dissolved in 170 mL of dry tetrahydrofuran (THF). The solution is cooled to -10 °C, and a tetrahydrofuran solution of methanesulfonic anhydride (Ms₂O, 7.7 g, 0.044 mol) is slowly added dropwise. After the addition is complete, the reaction is continued at -10 °C for 30 min. After the reaction is detected by LCMS, saturated sodium bicarbonate aqueous solution is added, and the mixture is extracted twice with ethyl acetate. The organic phases are combined, washed successively with saturated brine, dried over anhydrous sodium sulfate, filtered, and concentrated. The crude product is purified by column chromatography with dichloromethane:methanol (v:v) = 100:1 as the eluent, yielding 14.5 g of a white foamy solid, with a yield of 72.5%. [M+1]⁺= 545.2.

### (3) Synthesis of Compound 36

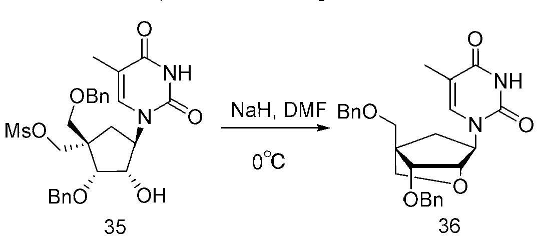

Under a nitrogen atmosphere, 60% sodium hydride (NaH, 3.2 g, 0.081 mol) is carefully added to 100 mL of dry N,N-dimethylformamide (DMF), and the mixture is cooled to 0 °C. A solution of compound 35 (14 g, 0.027 mol) in N,N-dimethylformamide is slowly added dropwise. After the addition is complete, the reaction is continued for 1 h. The reaction is detected by LCMS. After the reaction is complete, saturated ammonium chloride solution is added, and the mixture is extracted twice with ethyl acetate. The organic phases are combined, washed twice with saturated sodium chloride, dried over anhydrous sodium sulfate, filtered, and concentrated. The crude product is purified by column chromatography using dichloromethane:methanol (v:v) = 40:1 as the eluent, yielding 8.5 g of oily liquid, with a yield of 74%. [M+1]⁺=449.2.

### (4) Synthesis of compound I-1-1

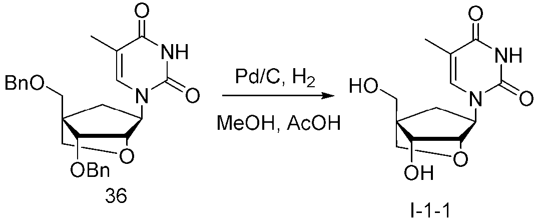

Compound 36 (4 g, 8.9 mmol) is dissolved in 40 mL of anhydrous methanol (MeOH), 50 µL of acetic acid (AcOH) and 400 mg of 10% palladium on carbon (Pd/C, 60% water content) are added, and the mixture is purged three times with hydrogen (H₂). The mixture is stirred overnight at room temperature. The reaction is monitored by LCMS until completion, filtered, and concentrated to give 2.2 g of white solid, 90% yield. [M+1]⁺=269.2;1H NMR (400 MHz, DMSO-d6) δ 11.27 (s, 1H), 7.43 (d, J = 1.3 Hz, 1H), 5.76 (s, 2H), 4.23 (dd, J = 9.7, 5.6 Hz, 1H), 3.91 (d, J = 14.6 Hz, 2H), 3.70 - 3.61 (m, 2H), 3.53 - 3.43 (m, 3H), 2.05 (dd, J = 13.6, 9.7 Hz, 1H), 1.81 - 1.78 (m, 3H), 1.78 - 1.72 (m, 1H).

### (5) Synthesis of Compound 37

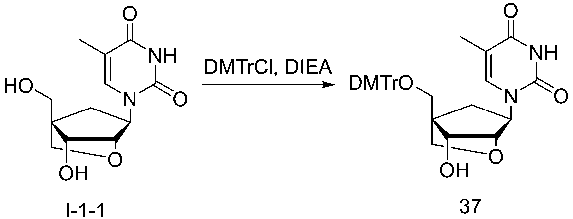

Under a nitrogen atmosphere, compound I-1-1 (1.6 g, 6.0 mmol) and 4,4'-bismethoxytriphenylmethyl chloride (DMTrCl, 4.65 g, 13.8 mmol) are dissolved in 30 mL of dry dichloromethane. The solution is cooled to 0 °C and N,N-diisopropylethylamine (DIEA, 2.3 g, 18 mmol) is slowly added dropwise. After the addition is complete, the mixture is stirred at room temperature for 2 h. The reaction is monitored by LCMS until complete. The organic phase is washed successively with water, then with saturated brine, dried over anhydrous sodium sulfate, filtered, and concentrated. The crude product is purified by column chromatography using dichloromethane:methanol (v:v) = 40:1 as the eluent, yielding 2.8 g of a pale yellow solid, with a yield of 86%. [M+1]⁺=571.2;1H NMR (400 MHz, DMSO-d6) δ 11.26 (s, 1H), 7.42 (d, J = 1.4 Hz, 1H), 7.38 - 7.28 (m, 4H), 7.22 (ddt, J = 6.9, 4.8, 2.2 Hz, 5H), 6.92 - 6.86 (m, 4H), 5.10 (d, J = 4.6 Hz, 1H), 4.30 (dd, J = 9.9, 5.8 Hz, 1H), 3.96 (d, J = 4.7 Hz, 1H), 3.91 (s, 1H), 3.74 (s, 6H), 3.67 (s, 2H), 3.29 (d, J = 9.0 Hz, 1H), 3.12 (d, J = 9.0 Hz, 1H), 2.30 (dd, J = 13.5, 9.8 Hz, 1H), 1.78 (d, J = 1.1 Hz, 4H).

### (6) Synthesis of compound I-1-5

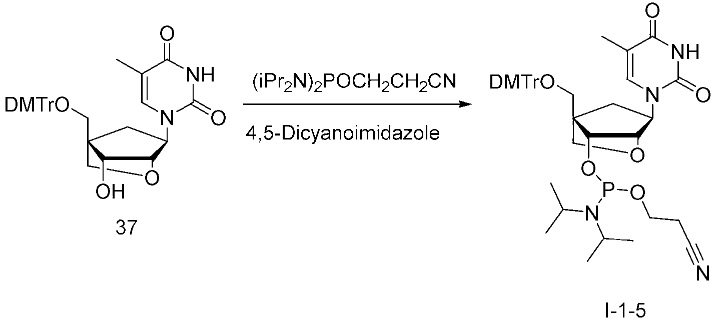

Under a nitrogen atmosphere, compound 37 (700 mg, 1.2 mmol) and 4,5-dicyanoimidazole (354 mg, 3 mmol) are dissolved in 10 mL of dry dichloromethane. Bis(diisopropylamino)(2-cyanoethoxy)phosphine ((iPr₂N₂)₂POCH₂CH₂CN, 904 mg, 3 mmol) are added dropwise at room temperature, and stirring is continued for 2 h. After the reaction is monitored by LCMS, dichloromethane is added, and the organic phase is washed sequentially with saturated brine, dried over anhydrous sodium sulfate, filtered, and concentrated. The crude product is separated using high presser liquid chromatography (HPLC) on a C18 column with a mobile phase of acetonitrile (containing 0.0035% diisopropylamine):water (containing 0.0035% diisopropylamine) (v:v) = 8:2, yielding 560 mg of white solid, 60% yield. [M+1]⁺=771.3;³¹PNMR:148.30,148.04.

### Embodiment 9 5: Synthesis and purification of oligonucleotide compound 1-12 modified with Carbocyclic nucleosides.

Using an AKTAOligoPilot Plus 100 oligonucleotide automated synthesizer, the target oligonucleotide compound is synthesized in a multi-step solid-phase process on a polystyrene solid support (PS resin) according to the oligonucleotide compound 1-12 sequence, including deprotection, coupling, capping, oxidation, and ammonolysis. All phosphoramidite monomers are dissolved in anhydrous acetonitrile (200 mM) and dried with molecular sieve (3A). 5-Ethylthio-1H-tetrazazole (ETT, 350 mM in acetonitrile) is used as the activating agent solution. The coupling time of commercially available MOE monomer and DNA monomer is 12 min, and the coupling time of phosphoramidite monomers^{cLNA}A, ^{cLNA}T, and^{cLNA}G, as well as phosphoramidite monomer C₁₆, is 30 min, with the first monomer being coupled twice. The phosphate ester bonds are constructed using an aqueous solution of iodine (50 mM); to introduce the phosphorothioate bonds, hydrogen yellow element (200 mM pyridine solution) is used. For unreacted active groups, a mixture of acetic anhydride, N-methylimidazole, pyridine, and acetonitrile (N-methylimidazole / acetonitrile (v:v) = 1:4; acetic anhydride / pyridine / acetonitrile (v:v:v) = 2:3:5) is used as a capping agent. After the reaction, the solid support is pyrolyzed in concentrated ammonia at 55 °C for 16 h, filtered, and the mother liquor is concentrated to obtain the crude product.

The crude oligonucleotide analog obtained is purified by ion-pair reversed-phase liquid chromatography. The chromatographic column is a NanoQ-15L 9.5mL (8×190 mm) ion exchange column, the flow rate is 2mL/min, the mobile phase A is 10mM sodium hydroxide solution, and the mobile phase B is 10mM sodium hydroxide and 2.0M sodium chloride solution. Gradient elution is performed, and the fractions that met the purity requirements are combined and collected. The fractions are then concentrated by ultrafiltration membrane and lyophilized to obtain oligonucleotide compound with the required purity.

Embodiment 6: Antisense inhibition of HBV by the tested oligonucleotide compound in HepG2.2.15 cells.

The design contains an antisense oligonucleotide compound targeting human HBV with a carbocyclic nucleoside modification, and a sequence without carbocyclic nucleoside modification is used as a control sequence. The modified sequence is as shown in Table 1. This compound served as a positive control, and its inhibitory effect on HBV activity in vitro is tested in HepG2.2.15 cells.

### Method:

On day 0, the test compound is first serially diluted with RNase-free water to eight concentrations (30.000 nM, 7.500 nM, 1.875 nM, 0.469 nM, 0.117 nM, 0.029 nM, 0.007 nM, 0.002 nM). HepG2.2.15 cells cultured in culture flasks are rinsed with DuPont phosphatebuffered saline (DPBS), digested with trypsin, and the cells are collected, counted, and adjusted to an appropriate density. Cells are seeded into 96-well plates at a density of 2.25 × 10⁴ cells per well. Simultaneously, the compound is transfected into the cells using Lipofectamine RNAiMAX reagent.

Day 3, replace with fresh culture medium.

On day 6, cell supernatant is collected, and the HBV DNA content in the supernatant is detected by real-time quantitative PCR (qPCR). The HBeAg and HBsAg content in the supernatant is detected by enzyme-linked immunosorbent assay (ELISA). Finally, cells are collected, RNA is extracted, and the total intracellular HBV RNA is detected by real-time quantitative reverse transcription PCR (RT-qPCR).

The test compound is transfected into HepG2.2.15 cells, and the anti-HBV activity of the test compound is evaluated by detecting HBV DNA, HBeAg, HBsAg in the cell supernatant and intracellular HBV RNA.

Eight concentration points of the test compound are measured in duplicate in two wells.

### Data statistics:

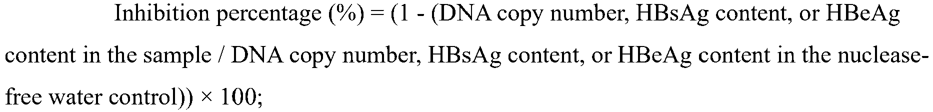

Inhibition percentage (%) = (1 - (DNA copy number, HBsAg content, or HBeAg content in the sample / DNA copy number, HBsAg content, or HBeAg content in the nuclease-free water control)) × 100; 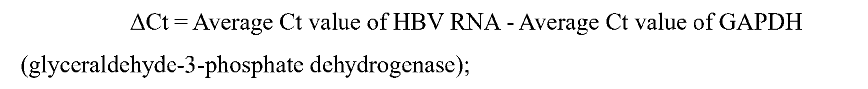 ΔCt = Average Ct value of HBV RNA - Average Ct value of GAPDH (glyceraldehyde-3-phosphate dehydrogenase); $ΔΔCt = ΔCt \left(sample\right) - ΔCt \left(nuclease-free water control\right) ;$

Relative amount of target gene mRNA = 2^{^-ΔΔCt}; 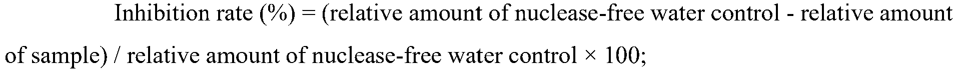 Inhibition rate (%) = (relative amount of nuclease-free water control - relative amount of sample) / relative amount of nuclease-free water control × 100; 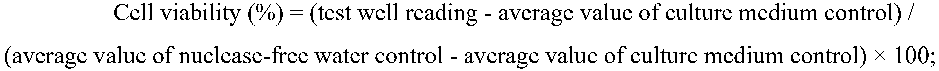 Cell viability (%) = (test well reading - average value of culture medium control) / (average value of nuclease-free water control - average value of culture medium control) × 100;

The IC₅₀ (half-maximal inhibitory concentration) and CC₅₀ (half-maximal toxic concentration) values of the compound are calculated using GraphPad Prism software. The curve fitting method is log(inhibition) vs. response--Variable slope.

### Results:

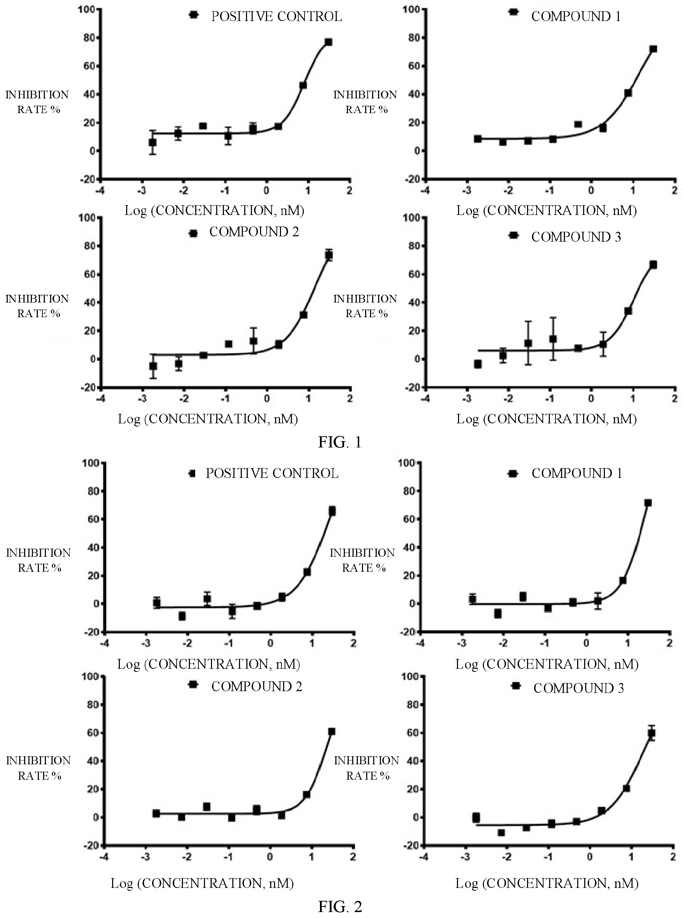

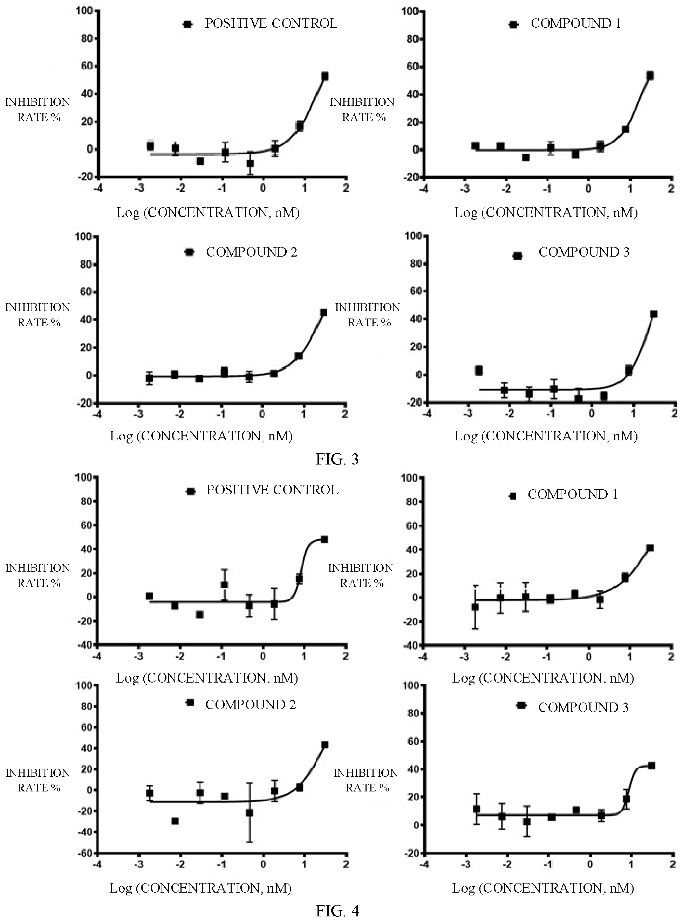

The results are as shown in Table 2, FIG. 1, FIG. 2, FIG. 3, and FIG. 4. FIG. 1 shows a fitted curve for HBV DNA inhibition rate of Embodiment 6, FIG. 2 shows a fitted curve for HBsAg inhibition rate of Embodiment 6, FIG. 3 shows a fitted curve for HBeAg inhibition of Embodiment 6, and FIG. 4 shows a fitted curve for HBV RNA inhibition rate of Embodiment 6. It can be seen that the IC50₅₀ values for inhibiting HBV DNA by the positive control, compound 1, compound 2, and compound 3 are 8.03 nM, 13.60 nM, 14.26 nM, and 10.39 nM, respectively; the IC50₅₀ for inhibiting HBsAg by the four compounds are 29.21 nM, 26.74 nM, 23.39 nM, and 19.59 nM, respectively, meaning that compounds 1 to 3 are more effective at inhibiting HBsAg than the positive control, where the IC50₅₀ for inhibiting HBeAg by the positive control and compound 1 are 25.65 nM and 17.88 nM, respectively, other compounds show inhibitory activity of less than 50% against HBeAg and HBsAg at the highest concentration. Compared with the positive control and other compounds, compound 1 is more effective in inhibiting HBeAg. All compounds show inhibitory activity of less than 50% against HBV RNA at the highest concentration (30 nM), with IC₅₀> 30 nM.

**Table 2 Anti-HBV activity of test compounds**

| Compounds | IC₅₀ (nM) | | | | CC₅₀(nM) |
|---|---|---|---|---|---|
| | HBV DNA | HBsAg | HBeAg | HBV RNA | |
| Positive Control | 8.03 | 29.21 | 25.65 | >30 | >30 |
| Compound 1 | 13.60 | 26.74 | 17.88 | >30 | >30 |
| Compound 2 | 14.26 | 23.39 | >30 | >30 | >30 |
| Compound 3 | 10.39 | 19.59 | >30 | >30 | >30 |

Embodiment 7: Antisense inhibition of HBV by test oligonucleotide compounds in HepG2.2.15 cells.

The design contains an antisense oligonucleotide compound targeting human HBV with carbon-cyclic nucleoside modification, the modified sequence is as shown in Table 1, and their inhibitory effect on HBV activity in vitro is tested in HepG2.2.15 cells.

Method: Same as Embodiment 6. The difference lies in the concentration of the test compound, which differs from that in Embodiment 6. The test compound is diluted to eight concentrations (270.000 nM, 90.000 nM, 30.000 nM, 10.000 nM, 3.333 nM, 1.111 nM, 0.3704 nM, 0.1235 nM).

Data statistics: Same as Embodiment 6.

### Results:

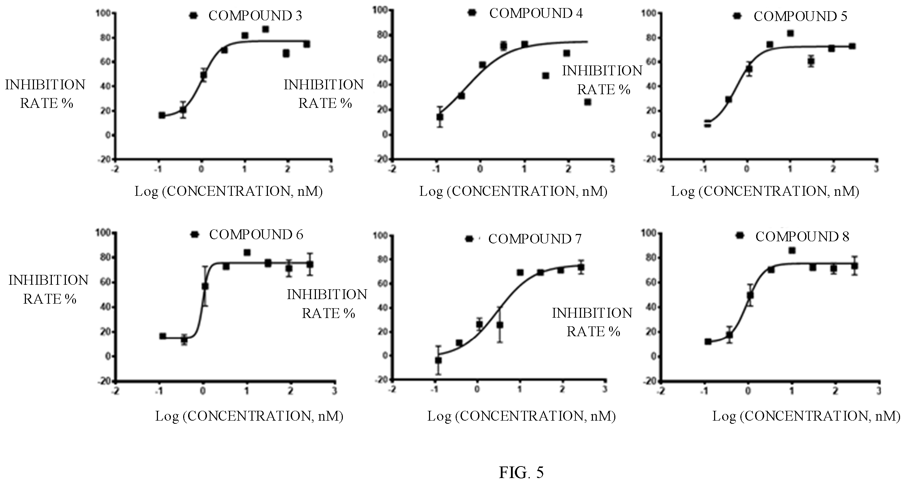

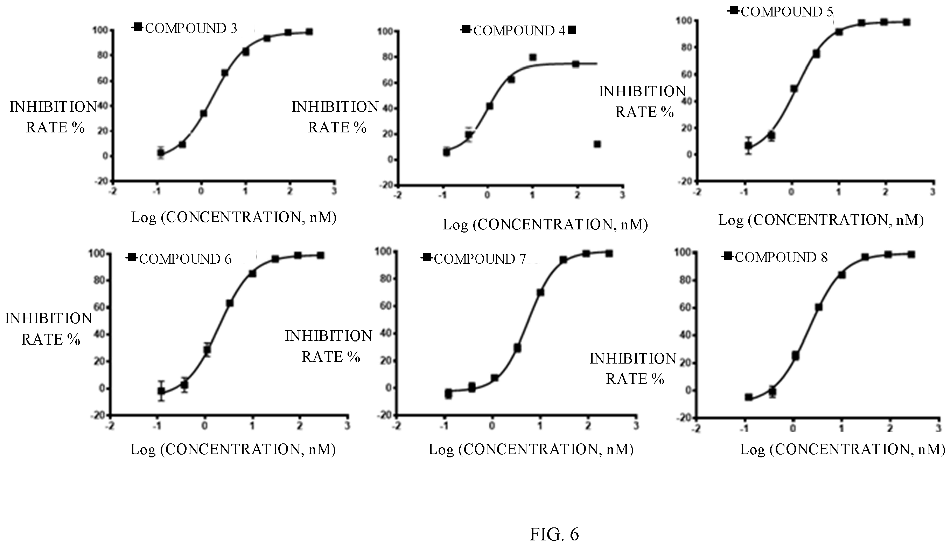

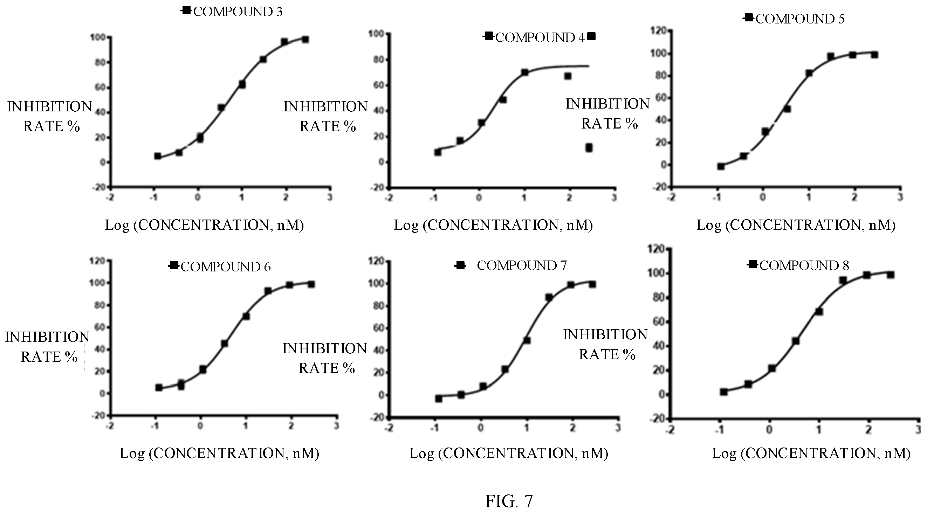

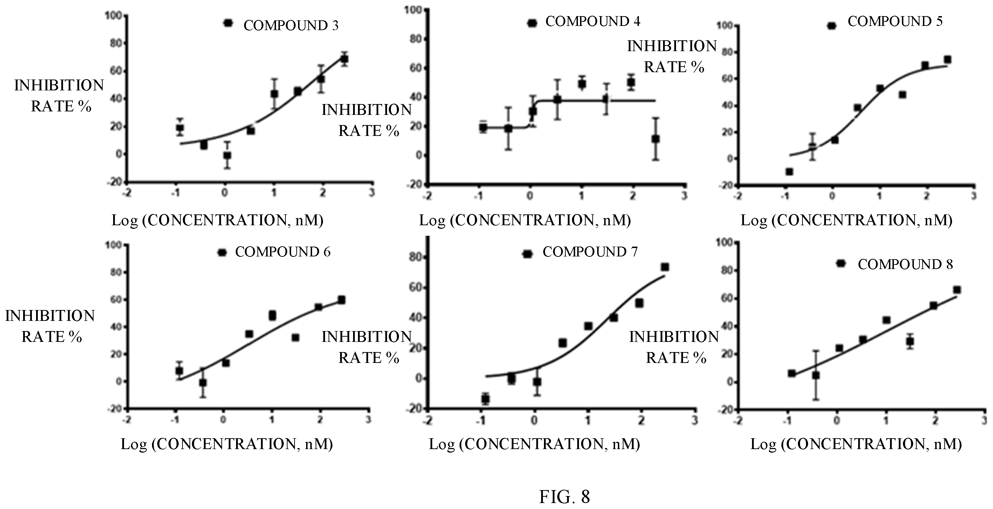

The results are as shown in Table 3, FIG. 5, FIG. 6, FIG. 7, and FIG. 8. In Table 3, NA indicates not detected. FIG. 5 shows a fitted curve for HBV DNA inhibition rate of Embodiment 7, FIG. 6 shows a fitted curve for HBsAg inhibition rate of Embodiment 7, FIG. 7 shows a fitted curve for HBeAg inhibition of Embodiment 7, and FIG. 8 shows a fitted curve for HBV RNA inhibition rate of Embodiment 7. It can be seen that all tested compounds, compounds 3 to 8, have inhibitory activity against HBV.

**Table 3 Anti-HBV activity of the test compound**

| Compounds | IC₅₀ (nM) | | | | CC₅₀(nM) |
|---|---|---|---|---|---|
| | HBV DNA | HBsAg | HBeAg | HBV RNA | |
| Compound 3 | 1.04 | 1.82 | 5.48 | 58.98 | 48.78 |
| Compound 4 | 0.46 | 1.01 | 2.05 | NA | NA |
| Compound 5 | 0.56 | 1.23 | 2.67 | 3.99 | 14.86 |
| Compound 6 | 0.99 | 1.99 | 4.46 | 3.18 | 23.97 |
| Compound 7 | 2.91 | 5.53 | 9.34 | 22.57 | 28.84 |
| Compound 8 | 0.94 | 2.12 | 4.47 | 10.73 | 23.01 |

Embodiment 8: Antisense inhibition of HBV by the test oligonucleotide compound in HepG2.2.15 cells.

The design contains an antisense oligonucleotide compound targeting human HBV with a carbocyclic nucleoside modification, and a sequence without carbocyclic nucleoside modification is used as a control sequence. The modified sequence is as shown in Table 1. This compound served as a positive control, and its inhibitory effect on HBV activity in vitro is tested in HepG2.2.15 cells.

Method: Same as Embodiment 7.

Data statistics: Same as Embodiment 6.

### Results:

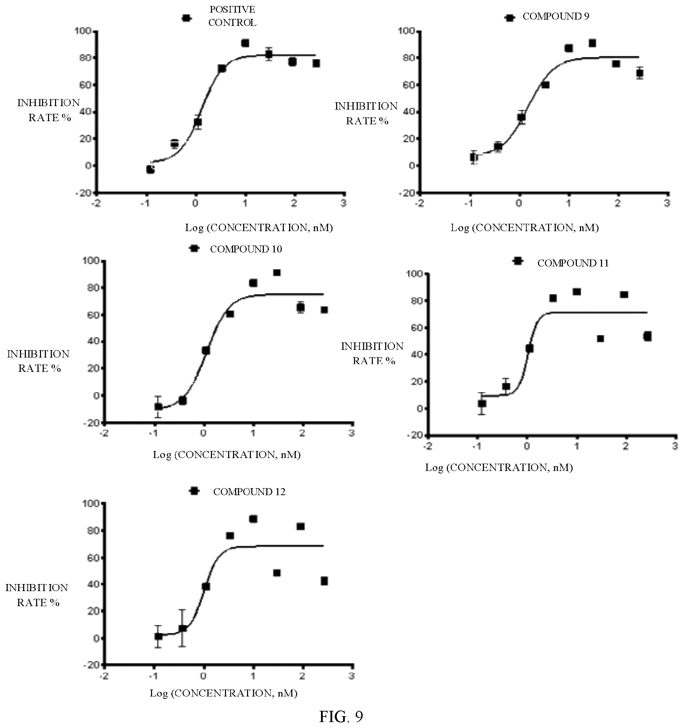

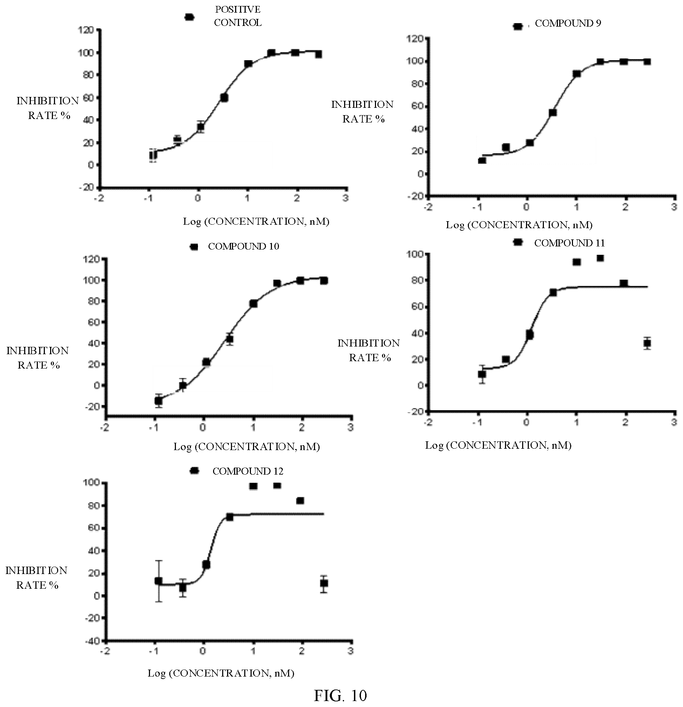

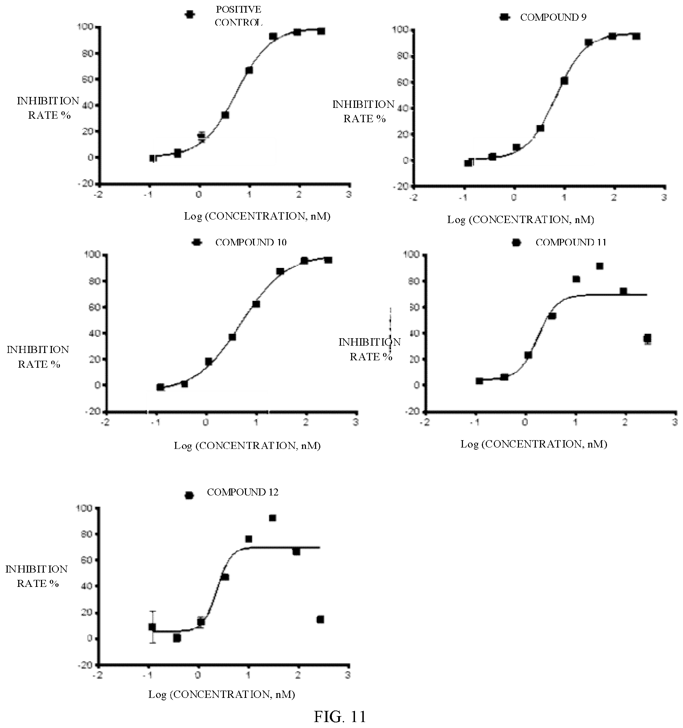

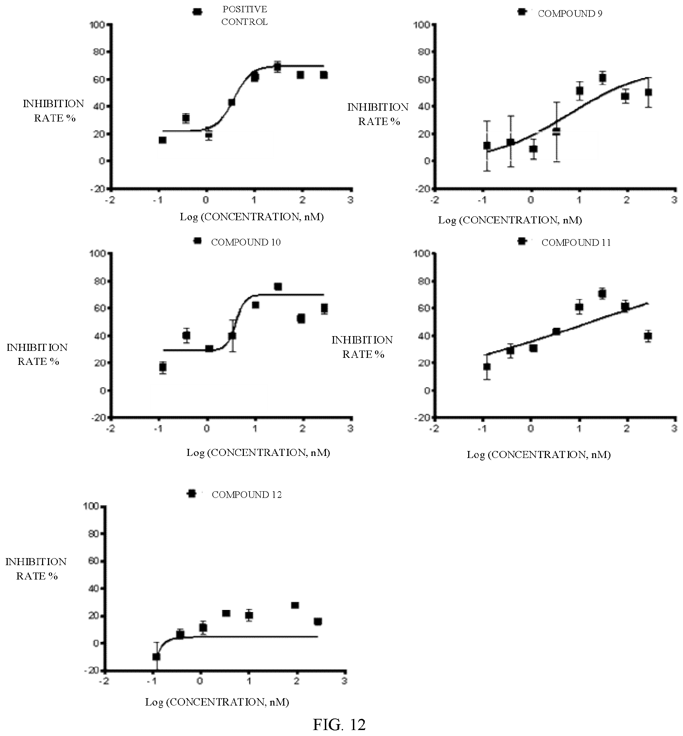

The results are as shown in Table 4, FIG. 9, FIG. 10, FIG. 11, and FIG. 12. In Table 4, NA indicates not detected. FIG. 9 shows a fitted curve for HBV DNA inhibition rate of Embodiment 8, FIG. 10 shows a fitted curve for HBsAg inhibition rate of Embodiment 8, FIG. 11 shows a fitted curve for HBeAg inhibition of Embodiment 8, and FIG. 12 shows a fitted curve for HBV RNA inhibition rate of Embodiment 8. It can be seen that compounds 9 to 12 have inhibitory activity against HBV.

**Table 4 Anti-HBV activity of the test compound**

| Compounds | IC₅₀(nM) | | | | CC₅₀(nM) |
|---|---|---|---|---|---|
| | HBsAg | HBeAg | HBV DNA | HBV RNA | |
| Positive Control | 2.60 | 5.50 | 1.31 | 3.99 | 37.20 |
| Compound 9 | 3.60 | 6.81 | 1.51 | 6.12 | 89.11 |
| Compound 10 | 2.66 | 4.94 | 1.14 | 5.05 | 40.22 |
| Compound 11 | 1.22 | 1.73 | 1.04 | 16.94 | NA |
| Compound 12 | 1.37 | 2.68 | 1.01 | >270 | NA |

Embodiment 9: Anti-HBV activity of the test oligonucleotide compound in C57BL/6-HBV transgenic model mice.

The design contains an antisense oligonucleotide compound targeting human HBV with a carbocyclic nucleoside modification, and a sequence without carbocyclic nucleoside modification is used as a control sequence. The modified sequence is as shown in Table 1. This compound served as a positive control, and its inhibitory effect on HBV activity in vivo is tested in C57BL/6-HBV transgenic mice.

Methods: Six-week-old female C57BL/6-HBV transgenic mice are acclimatized for 6 days. The day of the first administration is designated as day 0, the day before administration is day -1, the day after administration is day 1, and so on. Blood is collected from the submandibular venous plexus of all mice on day 3 prior to medicament administration. The collected blood samples are anticoagulated with K₂-EDTA (dipotassium ethylenediaminetetraacetate), centrifuged at 7000 g/min for 10 min at 4°C, and ~30 µL of plasma is collected for the detection of HBV DNA, HBsAg, and HBeAg levels in the plasma. HBV DNA levels in mouse plasma are quantitatively detected by qPCR, and HBsAg and HBeAg levels are quantitatively detected by ELISA. All mice are grouped according to their plasma HBV DNA, HBsAg, and HBeAg levels and body weight on day 3. Mice with low HBV DNA, HBsAg, and HBeAg levels and lower body weight are removed from the experiment. The selected 24 mice are evenly distributed into groups of 3, ensuring that there are no statistically significant differences in HBV DNA, HBsAg, HBeAg levels, and body weight among the groups on day 3 (P>0.05).

All experimental groups are administered the medicament subcutaneously (either physiological saline or the compounds listed in Table 1). Except for the physiological saline group, all other groups received a single dose of 45 mpk. Blood is collected from the submandibular venous plexus of mice on days 7, 14, and 21 after administration. The collected blood samples are anticoagulated with K₂-EDTA, centrifuged at 4°C and 7000 g/min for 10 min, and ~30 µL of plasma is collected for detecting HBV DNA, HBsAg, and HBeAg levels. On day 21, all mice are euthanized by CO₂inhalation. HBV DNA levels in mouse plasma are quantitatively detected by qPCR, and HBsAg and HBeAg levels are quantitatively detected by ELISA.

### Data statistics:

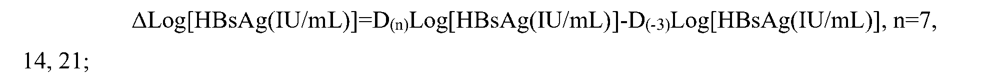

ΔLog[HBsAg(IU/mL)] = D(n)Log[HBsAg(IU/mL)] - D(-3)Log[HBsAg(IU/mL)], n = 7, 14, 21; 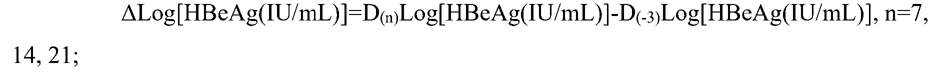 ΔLog[HBeAg(IU/mL)] = D(n)Log[HBeAg(IU/mL)] - D(-3)Log[HBeAg(IU/mL)] , n = 7, 14, 21; 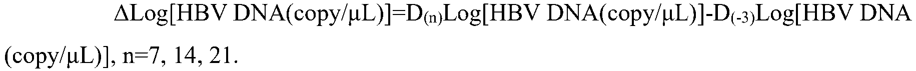 ΔLog[HBV DNA(copy/µL)]=D(n)Log[HBV DNA(copy/µL)]-D(-3)Log[HBV DNA (copy/µL)], n=7, 14, 21.

### Results:

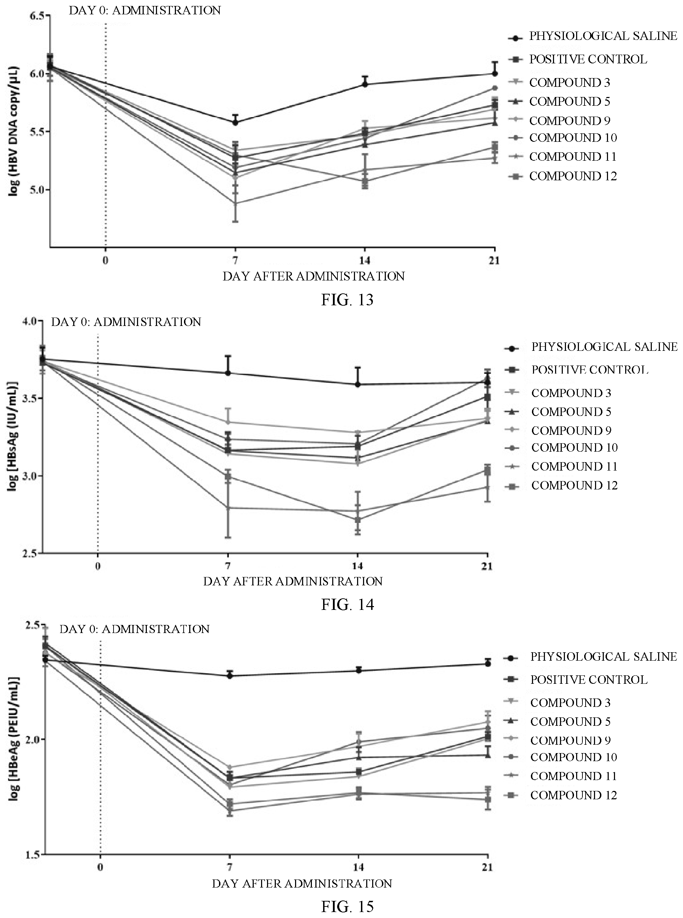

Table 5, FIG. 13, FIG. 14, and FIG. 15 show the changes and trends of Log values for HBV DNA, HBsAg, and HBeAg levels on days 7, 14, and 21 after administration to C57BL/6-HBV transgenic mice compared to before administration (day -3). Data are presented as decreases in Log values compared to before administration (day -3), including: ΔLog[HBsAg (IU/mL)], ΔLog[HBeAg (PEIU/mL)], and ΔLog[HBV DNA (copy/µL)]. It can be seen that, compared with the physiological saline group, the Log[HBsAg(IU/mL)], Log[HBeAg(PEIU/mL)], and Log[HBV DNA(copy/µL)] are significantly reduced on days 7, 14, and 21 after administration of the test compounds in the other groups. At the same time, compared with the positive control, the Log[HBsAg(IU/mL)], Log[HBeAg(PEIU/mL)], and Log[HBV DNA(copy/µL)] are significantly reduced in the compound 3, compound 5, compound 11, and compound 12 groups, especially in compound 11 and compound 12.

**Table 5 Anti-HBV activity of the test compound in transgenic mice**

| Compounds | ΔLog[HBsAg(IU/mL)] | | | ΔLog[HBeAg(PEIU/mL)] | | | ΔLog[HBV DNA(copy/µL)] | | |
|---|---|---|---|---|---|---|---|---|---|
| | D7 | D14 | D21 | D7 | D14 | D21 | D7 | D14 | D21 |
| Physiological saline | -0.09 | -0.16 | -0.15 | -0.07 | -0.05 | -0.02 | -0.49 | -0.16 | -0.06 |
| Positive Control | -0.56 | -0.54 | -0.22 | -0.57 | -0.54 | -0.39 | -0.8 | -0.59 | -0.34 |
| Compound 3 | -0.6 | -0.67 | -0.38 | -0.62 | -0.56 | -0.4 | -0.72 | -0.59 | -0.37 |
| Compound 5 | -0.59 | -0.63 | -0.39 | -0.59 | -0.5 | -0.49 | -0.91 | -0.67 | -0.48 |
| Compound 9 | -0.39 | -0.46 | -0.37 | -0.49 | -0.4 | -0.3 | -0.95 | -0.52 | -0.43 |
| Compound 10 | -0.5 | -0.52 | -0.10 | -0.58 | -0.39 | -0.33 | -0.86 | -0.6 | -0.17 |
| Compound 11 | -0.95 | -0.97 | -0.81 | -0.66 | -0.58 | -0.58 | -1.17 | -0.88 | -0.77 |
| Compound 12 | -0.76 | -1.03 | -0.71 | -0.69 | -0.64 | -0.67 | -0.75 | -0.98 | -0.69 |

Note: "Δ" represents the decrease compared to before administration; all Log values are logarithmic values with base 10.

As can be seen from the above, the oligonucleotide compound provided in the disclosure can reduce the expression levels of HBV RNA, HBV DNA and related proteins, and has excellent anti-HBV activity, and can be used in the prevention and treatment of HBV and HDV related diseases or symptoms.

The specific embodiments have been described in detail above, but the present disclosure is not limited to the specific embodiments described above. Those skilled in the art can make various modifications under the guidance of the disclosure without departing from the scope of protection of the disclosure, and these modifications all fall within the scope of protection of the present disclosure.

## Claims

1. An oligonucleotide compound, wherein the oligonucleotide compound comprises at least one carbocyclic nucleoside of formula (I), a nucleic acid base sequence of each of the at least one oligonucleotide compound is as shown in sequence identity 1, SEQ ID NO.1, 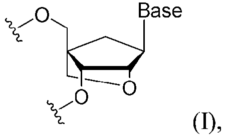 wherein Base is a heterocyclic base, 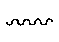 indicates a linking site.

2. The oligonucleotide compound of claim 1, wherein the oligonucleotide compound further comprises a lipophilic group, and the lipophilic group is located at at least one of a 5' end and a 3' end of the oligonucleotide compound.

3. The oligonucleotide compound of claim 2, wherein the lipophilic group is derived from a lipophilic compound having 4-32 carbon atoms;
the lipophilic compound comprises at least one member selected from a group consisting of alkane, an alkanoyl compound, a carboxyl compound, a retinyl compound, cholestene, cholesterol, dihydrotestosterone, hexadecylglycerol, menthol, borneol, a dimethoxytriphenylmethyl compound, a geranyloxyhexyl compound, and phenoxazine.

4. The oligonucleotide compound of claim 2, wherein the oligonucleotide compound further comprises a linker, the linker is linked to the lipophilic group, the linker comprises a biologically cleavable group.

5. The oligonucleotide compound of claim 4, wherein the linker further comprises a linking group, and the linking group links the biologically cleavable group and the lipophilic group; and
the linking group comprises at least one member selected from a group consisting of groups as shown in formulas (II-1) to (II-5),

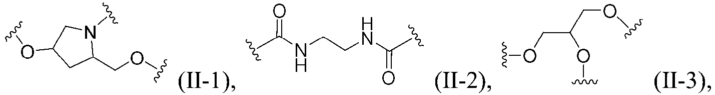

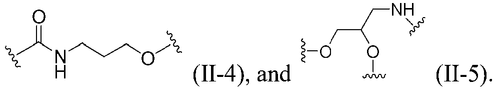

6. The oligonucleotide compound according to claim 5, wherein a combination of the linker and the lipophilic group comprises at least one member selected from a group consisting of groups as show in formulas (III-1) to (III-4), 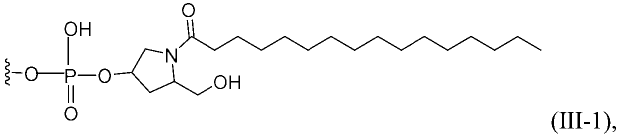 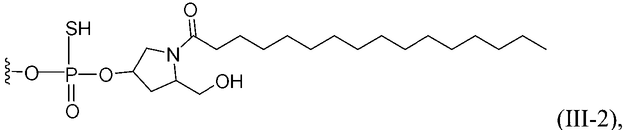 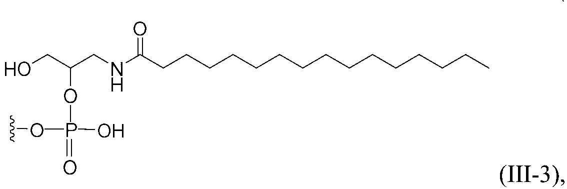 and

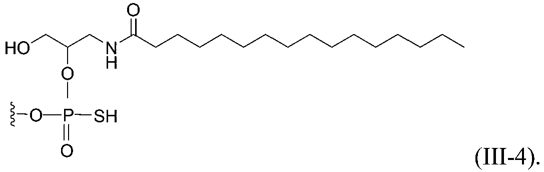

7. The oligonucleotide compound of claim 1, wherein the oligonucleotide compound consists of a 5' flanking segment, a intergenic segment, and a 3' flanking segment, a bases of the 5' flanking segment comprises GCAGA, a bases of the intergenic segment comprises GGTGAAGCGA, a bases of the 3' flanking segment comprises AGTGC; and all cytosines are 5-methylcytosine; nucleosides in the 5' flanking segment and the 3' flanking segment are modified with 2'-O-methoxyethyl, the 5' flanking segment and/or the 3' flanking segment contain the carbocyclic nucleoside, and adjacent nucleosides are linked by a phosphorothioate bond.

8. A medicament comprising the oligonucleotide compound of any one of claims 1 to 7 or a pharmaceutically acceptable salt thereof.

9. The medicament of claim 8, wherein the oligonucleotide compound or the pharmaceutically acceptable salt thereof constitutes the medicament as a single active ingredient or with another pharmaceutically acceptable active ingredient.

10. An oligonucleotide compound of any one of claims 1 to 7 or a pharmaceutically acceptable salt thereof, or the medicament of claim 8 or 9, for use as a medicament.

11. An oligonucleotide compound as defined in any one of claims 1 to 7 or a pharmaceutically acceptable salt thereof, or a medicament as defined in any of claims 8 or 9, for use in the prevention and/or treatment of HBV and/or HDV-related diseases or symptoms.
